# EUROPEAN PATENT APPLICATION

(11) **EP 4 685 155 A1**
(43) Date of publication of application: **28.01.2026**
(21) Application number: 24774234.9
(22) Date of filing: 21.03.2024
(51) Int. Cl.: C07K 14/55, C07K 16/28, A61K 39/00

(54) **PREPARATION CONTAINING ANTI-PD-1/MUTANT IL-2 IMMUNOCONJUGATE**

(30) Priority: 21.03.2023 CN 202310276058
(71) Applicant: Fortvita Biologics (Singapore) Pte. Ltd., Singapore 189767 (SG)
(72) Inventor: YU, Fang, Suzhou, Jiangsu 215123 (CN); XIE, Ruixia, Suzhou, Jiangsu 215123 (CN); HE, Bing, Suzhou, Jiangsu 215123 (CN)
(74) Representative: Sagittarius IP
(86) International application number: PCT/CN2024/083130
(87) International publication number: WO 2024/193670

(57) **Abstract**

The present invention relates to formulations comprising an immunoconjugate of a recombinant anti-programmed death receptor 1 (PD-1) antibody and a mutant interleukin-2 (IL-2) (also referred to as "αPD-1/IL2m"), particularly stable liquid formulations, as well as methods for preparing said formulations, and the use of said formulations for the treatment and/or prevention of diseases.

## Description

### TECHNICAL FIELD

The present invention relates to the field of immunoconjugate formulations. More specifically, the present invention relates to formulations comprising an immunoconjugate of a recombinant anti-programmed death receptor 1 (PD-1) antibody and a mutant interleukin-2 (IL-2) (hereinafter also referred to as "αPD-1/IL2m"), particularly stable liquid formulations, as well as methods for preparing said formulations, and the use of said formulations for the treatment and/or prevention of diseases.

### BACKGROUND ART

Interleukin-2 (IL-2), also known as T-cell growth factor (TCGF), is a multifunctional cytokine primarily produced by activated T cells, especially CD4+ T helper cells. Through binding to IL-2 receptors on different cells, IL-2 mediates multiple effects in immune responses. On the one hand, IL-2 has immune system-stimulating effects, promoting the proliferation and differentiation of T cells and natural killer (NK) cells. Therefore, IL-2 has been approved as an immunotherapeutic agent for the treatment of cancer and chronic viral infections. On the other hand, IL-2 can also promote the maintenance of immunosuppressive CD4+CD25+ regulatory T cells (i.e., Treg cells) (Fontenot et al., Nature Immunol 6, 1142-51 (2005); D'Cruz and Klein, Nature Immunol 6, 1152-59 (2005); Maloy and Powrie, Nature Immunol 6, 1171-72 (2005)), leading to immune suppression in patients caused by activated Treg cells.

Programmed death protein-1 (PD-1) is an important immune checkpoint protein, a 55 kDa type I transmembrane protein, primarily induced to express on the surface of activated T cells, and also expressed on B cells, NK cells, monocytes, DC cells, etc. Two cell surface glycoprotein ligands of PD-1 have been identified, namely programmed death-ligand 1 (PD-L1) and programmed death-ligand 2 (PD-L2). The ligands of PD-1 are highly expressed on many cancer cells. The binding of PD-1 to its ligands can lead to T cell apoptosis, immune unresponsiveness, T cell "exhaustion", and secretion of IL-10, etc. Therefore, blocking the PD-1 pathway can restore T cell function in cancer patients (Sheridan, Nature Biotechnology 30 (2012), 729-730). Monoclonal antibodies targeting PD-1 have been documented, such as Nivolumab from Bristol-Myers Squibb (BMS) and Pembrolizumab from Merck. Nivolumab (trade name OPDIVO^{®}) is a fully humanized IgG4 antibody molecule, and Pembrolizumab (trade name KEYTRUDA^{®}) is a humanized IgG4 antibody molecule. The anti-PD-1 monoclonal antibody, upon binding to PD-1 on T lymphocytes, can inhibit the binding of PD-1 to its ligands PD-L1 and PD-L2, thereby promoting T lymphocyte activation, proliferation, and production of immune-activating cytokines, and relieving PD-1-mediated suppression of immune surveillance by T lymphocytes with anti-tumor activity.

Given the importance of IL-2 and PD-1 in regulating immune responses, the inventors have obtained an anti-PD-1/IL-2 mutant protein immunoconjugate targeting both PD-1 and the IL-2 receptor through diligent research (see Patent Application No. PCT/CN2022/120265, the entire contents of which are incorporated herein by reference). The immunoconjugate exhibits enhanced anti-tumor effects while also showing reduced side effects, making it a promising candidate for clinical tumor treatment.

For clinical medications, drug stability is one of the critical indicators to ensure drug efficacy and safety. Obtaining a formulation that confers good stability to the drug is a key factor for maintaining the drug's safety and efficacy during its shelf life. Therefore, to meet the requirements for drug stability, there remains a need in the field for further targeted development of pharmaceutical formulations suitable for anti-PD-1/IL-2 mutant protein immunoconjugates.

### SUMMARY OF THE INVENTION

To address the above issues, the inventors of the present invention designed formulation screening experiments for the recombinantly expressed anti-PD-1/IL-2 mutant protein immunoconjugate. Through high-temperature experiments, the pH of the buffer solution, buffer system, and excipients were screened; and the formulations were evaluated and confirmed based on indicators such as appearance, visible particulates, protein content, pH, purity, charge heterogeneity, polysorbate-80 content, and biological activity. Ultimately, the liquid formulation of the present invention was successfully developed. The formulation of the present invention not only ensures the quality control of the product during its shelf life but can also be prepared through a stable and easily achievable sterile formulation process.

Therefore, in a first aspect, the present invention provides a liquid formulation comprising an αPD-1/IL2m immunoconjugate. In some embodiments, the liquid formulation of the present invention is an injectable preparation, preferably an intravenous infusion solution.

In some embodiments, the liquid formulation of the present invention comprises:
(i) αPD-1/IL2m immunoconjugate;
(ii) a buffering agent;
(iii) a stabilizer; and
(iv) a surfactant,
wherein the liquid formulation has a pH of about 5.0 to about 6.5, such as about 5.0 to about 5.5, preferably about 5.0.

In some embodiments, the αPD-1/IL2m immunoconjugate according to the present invention comprises: (a) an anti-PD-1 half-antibody and (b) a mutant IL2 fusion polypeptide,
wherein the anti-PD-1 half-antibody comprises an Fab fragment fused to an Fc region and comprises three heavy chain CDR sequences contained in the heavy chain variable region sequence of SEQ ID NO: 8; and three light chain CDR sequences contained in the light chain variable region sequence of SEQ ID NO: 15; and
wherein the mutant IL2 fusion polypeptide comprises a mutant IL-2 polypeptide fused to an Fc region, and the mutant IL-2 polypeptide comprises (i) N88R + S130R; and (ii) a B'C' loop sequence of AGDASIH or AQSKNFH, and optionally (iii) T3A.

In some embodiments, the liquid formulation comprises about 0.5 mg/mL to about 150 mg/mL of the immunoconjugate, preferably, the concentration of the immunoconjugate is about 0.5 mg/mL to about 5 mg/mL.

In some embodiments, the buffering agent is selected from the group consisting of: histidine buffering agent, glutamate buffering agent, phosphate buffering agent, acetate buffering agent, citrate buffering agent, tris(hydroxymethyl)aminomethane (tris) buffering agent, and combinations thereof. Preferably, the liquid formulation according to the present invention comprises a histidine buffering agent. In some embodiments, in the liquid formulation according to the present invention, the (total) concentration of the buffering agent is about 0.5 mM to about 200 mM, about 5 mM to about 50 mM, or about 5 mM to about 20 mM.

In some embodiments, the stabilizer is selected from the group consisting of: sugars (e.g., sucrose, dextrose, lactose, maltose, trehalose, cyclodextrin, maltodextrin, dextran), amino acids (e.g., methionine, arginine, and salts thereof), or combinations thereof. Preferably, the stabilizer comprises sucrose, methionine, or a combination thereof. In some embodiments, the (total) concentration of the stabilizer is about 1 mM to about 1000 mM, or about 50 mM to about 500 mM.

In some embodiments, the surfactant is selected from the group consisting of: polysorbate surfactants, such as polysorbate 80 and polysorbate 20, preferably, the surfactant is polysorbate 80. In some embodiments, the (total) concentration of the surfactant is about 0.01 mg/mL to about 10 mg/mL, about 0.05 mg/mL to about 5 mg/mL, about 0.1 mg/mL to about 5 mg/mL, or about 0.1 mg/mL to about 1 mg/mL.

In some embodiments, the liquid formulation of the present invention comprises:
(i) an αPD-1/IL2m immunoconjugate;
(ii) a histidine buffering agent;
(iii) sucrose and methionine; and
(iv) polysorbate-80,

and optionally (v) EDTA,
wherein the liquid formulation has a pH of about 5.0 to about 6.5, such as about 5.0 to about 5.5, preferably about 5.0.

In some embodiments, the liquid formulation of the present invention comprises:
(i) an αPD-1/IL2m immunoconjugate;
(ii) histidine and histidine hydrochloride;
(iii) sucrose and methionine; and
(iv) polysorbate-80,

and optionally (v) EDTA,
wherein the liquid formulation has a pH of about 5.0 to about 6.5, such as about 5.0 to about 5.5, preferably about 5.0.

Preferably, the liquid formulation of the present invention comprises:
(i) about 1 mg/mL to about 10 mg/mL of αPD-1/IL2m immunoconjugate;
(ii) about 10 mM to about 30 mM of histidine buffering agent;
(iii) about 70 mg/mL to about 90 mg/mL of sucrose, or a combination of about 50 mg/mL to about 80 mg/mL of sucrose and about 40 mM to about 60 mM of methionine; and
(iv) about 0.2 mg/mL to about 0.4 mg/mL of polysorbate 80;
   wherein the pH of the liquid formulation is about 5.0-5.5, preferably about pH 5.0;
   or, the liquid formulation comprises
      (i) about 1.0 mg/mL of αPD-1/IL2m immunoconjugate;
      (ii) about 10 mM of histidine buffering agent;
      (iii) about 80 mg/mL of sucrose, or a combination of about 70 mg/mL of sucrose and about 50 mM of methionine, and
      (iv) about 0.3 mg/mL of polysorbate 80;
   wherein the pH of the liquid formulation is about 5.0-5.5, preferably about pH 5.0. In any of the above embodiments, preferably, the histidine buffering agent is a buffering system comprising histidine and its salts, particularly a histidine buffering system comprising histidine and histidine hydrochloride.

In some more preferred embodiments, the liquid formulation of the present invention comprises:
(i) about 1.0 mg/mL of αPD-1/IL2m immunoconjugate;
(ii) about 0.12 mg/mL of histidine and about 1.94 mg/mL of histidine hydrochloride;
(iii) about 70 mg/mL of sucrose and about 7.49 mg/mL of methionine, and
(iv) about 0.3 mg/mL of polysorbate 80;
wherein the pH of the liquid formulation is about 5.0-5.5, preferably about pH 5.0.

In a second aspect, the present invention provides a solid formulation obtained by solidifying the liquid formulation of the present invention. The solidification process is carried out by, for example, crystallization, spray drying, or lyophilization. In a preferred embodiment, the solid formulation is, for example, in the form of a lyophilized powder for injection. The solid formulation can be reconstituted in a suitable vehicle before use to form a reconstituted formulation of the present invention. The reconstituted formulation is also a liquid formulation of the present invention. In one embodiment, the suitable vehicle is selected from water for injection, organic solvents for injection, including but not limited to oils for injection, ethanol, propylene glycol, or combinations thereof.

In a third aspect, the present invention provides a pharmaceutical container comprising the liquid formulation of the present invention. In some embodiments, the container is an injection container.

In a fourth aspect, the present invention provides a delivery device comprising the liquid formulation or solid formulation of the present invention. In one embodiment, the delivery device of the present invention is provided in the form of a prefilled syringe containing the liquid formulation or solid formulation of the present invention, for example, for intravenous, subcutaneous, intradermal or intramuscular injection, or intravenous infusion.

In a fifth aspect, the present invention provides a method for delivering αPD-1/IL2m immunoconjugate to a subject, such as a mammal, comprising administering to the subject the liquid formulation or solid formulation of the present invention, wherein the delivery is implemented, for example, by using a delivery device such as a prefilled syringe.

In a sixth aspect, the present invention provides use of the liquid formulation or solid formulation of the present invention for treating or preventing cancer in a subject; or for preparing a medicament or delivery device (e.g., a prefilled syringe) for treating or preventing cancer in a subject. Preferably, the cancer is a solid tumor or a hematologic tumor, such as gastrointestinal tumor or melanoma, for example, colorectal cancer or colon cancer; for example, the cancer is a PD-1 antibody therapy-resistant cancer.

Other embodiments of the present invention will become apparent by reference to the detailed description that follows.

### BRIEF DESCRIPTION OF THE DRAWINGS

The preferred embodiments of the present invention described in detail below will be better understood when read in conjunction with the accompanying drawings. For the purpose of illustrating the present invention, the drawings show presently preferred embodiments. However, it should be understood that the present invention is not limited to the precise arrangements and means shown in the figures.

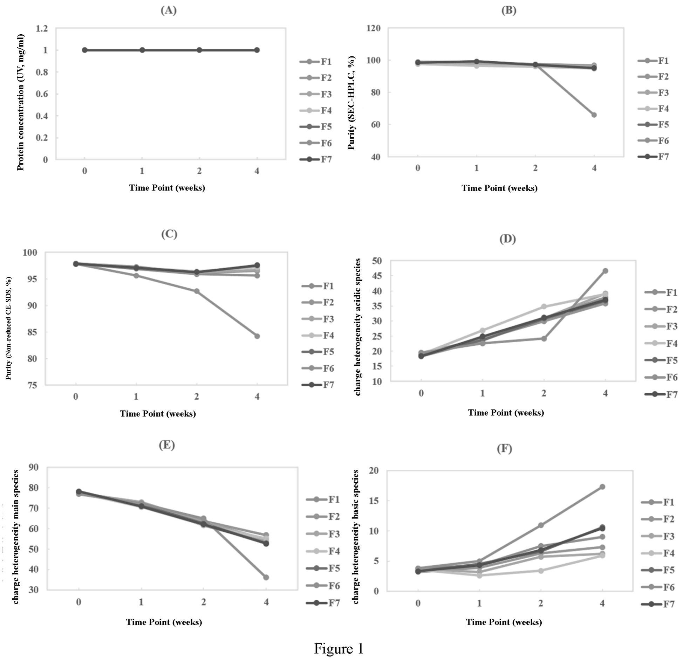

Figure 1 shows trend charts of formulation study results. (A) Trend chart of protein content (UV method); (B) Trend chart of purity (SEC-HPLC method); (C) Trend chart of purity (nrCE-SDS method); (D) Trend chart of charge heterogeneity - acidic species (iCIEF method); (E) Trend chart of charge heterogeneity - main species (iCIEF method); (F) Trend chart of charge heterogeneity - basic species (iCIEF method).

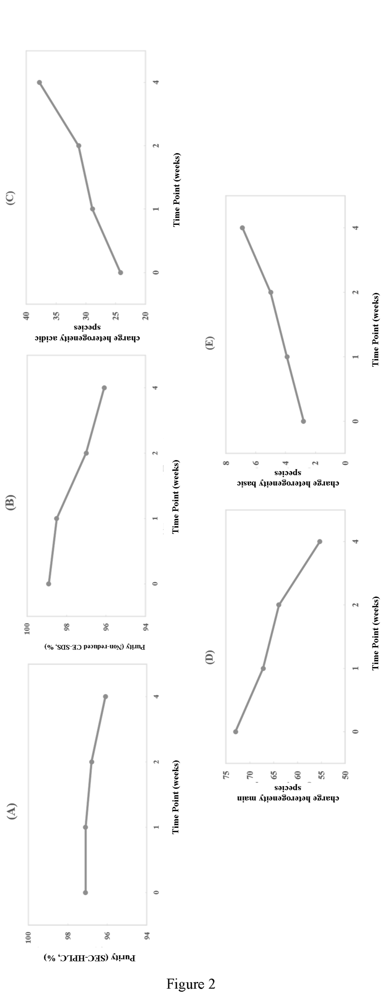

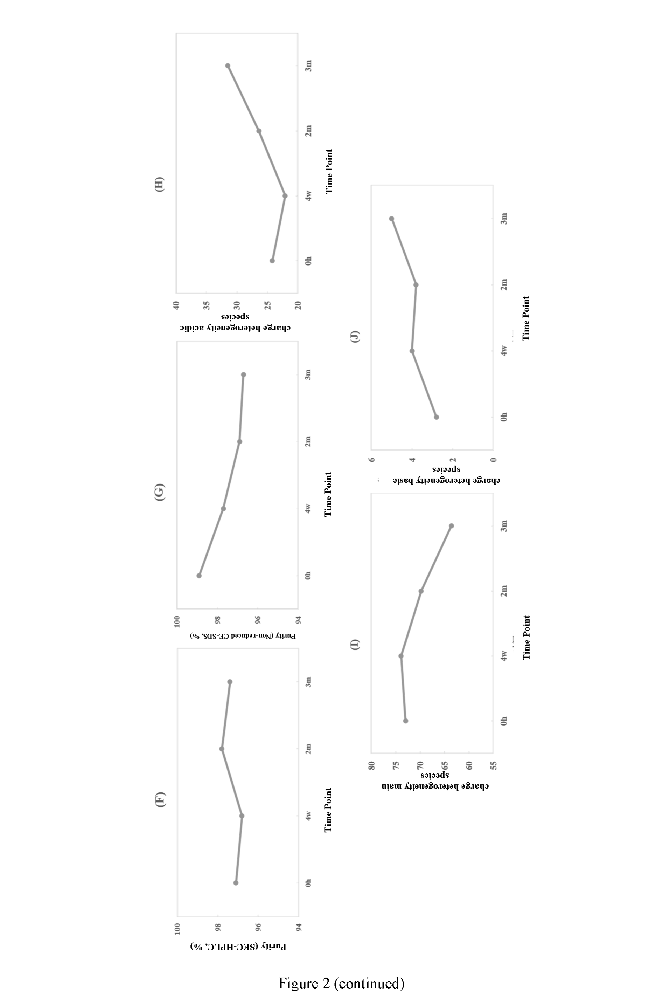

Figure 2 shows trend charts of formulation confirmation study results. (A)-(E) show stability study results at 40°C, and (F)-(J) show stability study results at 25°C. (A) and (F) show trend charts of purity (SEC-HPLC method); (B) and (G) show trend charts of purity (nrCE-SDS method); (C) and (H) show trend charts of charge heterogeneity - acidic species (iCIEF method); (D) and (I) show trend charts of charge heterogeneity - main species (iCIEF method); (E) and (J) show trend charts of charge heterogeneity - basic species (iCIEF method).

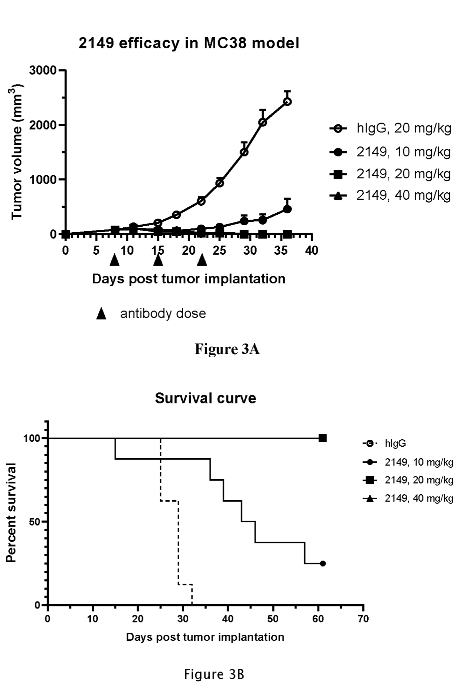

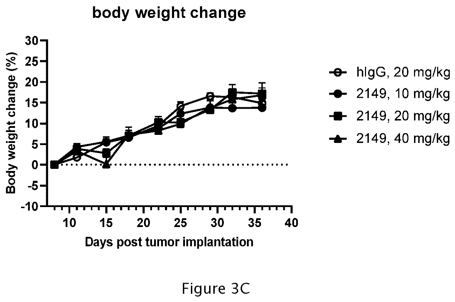

Figure 3 shows the in vivo efficacy of immunoconjugate 2149 protein. (A) shows the tumor volume change curve reflecting the antitumor effect of 2149 on MC38 tumors in mice; (B) shows the survival curve reflecting the antitumor effect of 2149 on MC38 tumors in mice; (C) shows the effect of 2149 on mouse body weight.

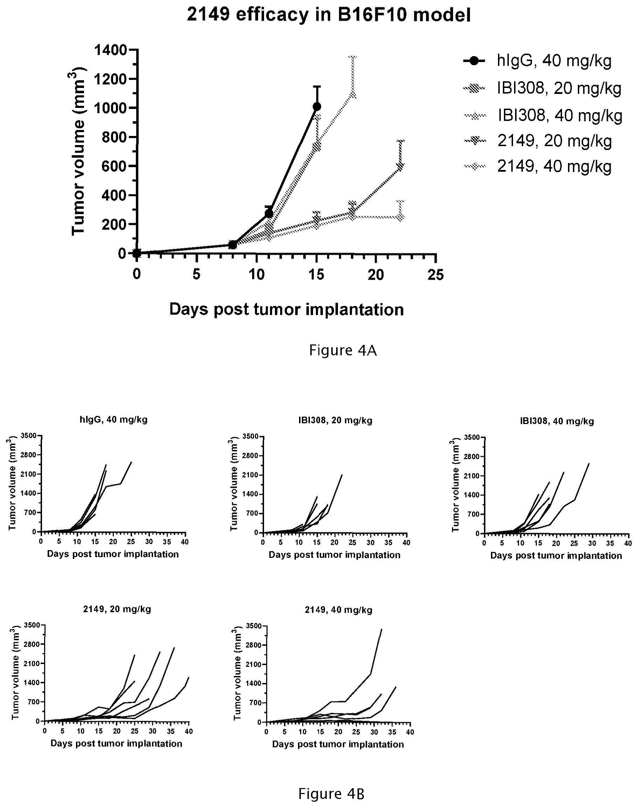

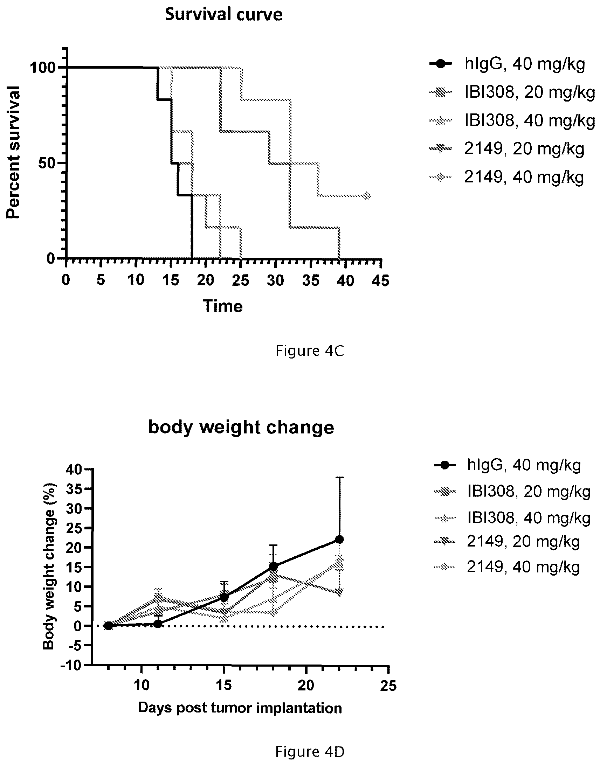

Figure 4 shows the in vivo efficacy of immunoconjugate 2149 protein. (A) shows the tumor volume change curve reflecting the antitumor effect of 2149 on B16F10 tumors in mice; (B) shows the individual tumor volume change curves reflecting the antitumor effect of 2149 on B16F10 tumors in mice; (C) shows the survival curve reflecting the antitumor effect of 2149 on B16F10 tumors in mice; (D) shows the effect of 2149 on mouse body weight.

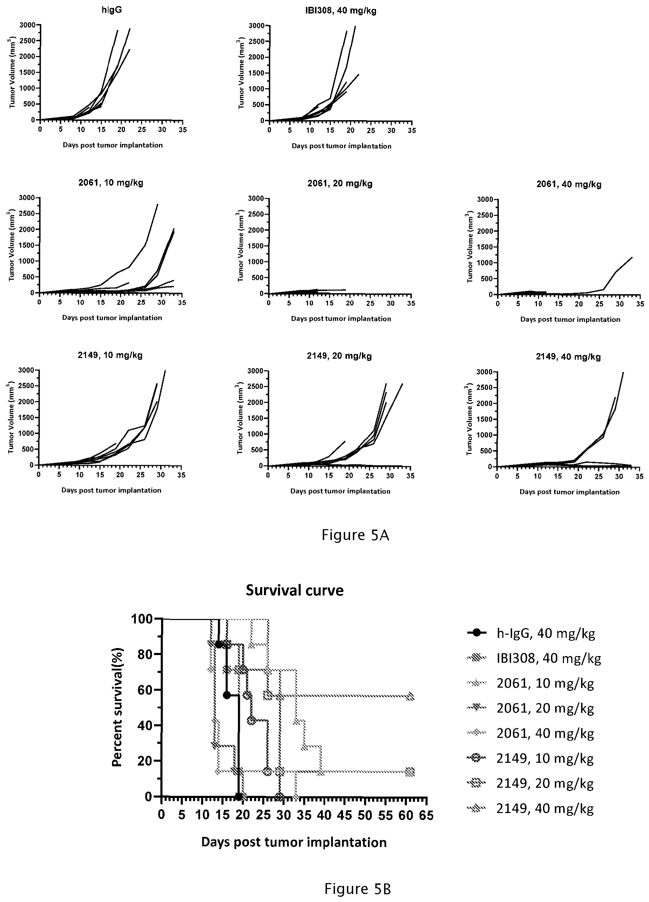

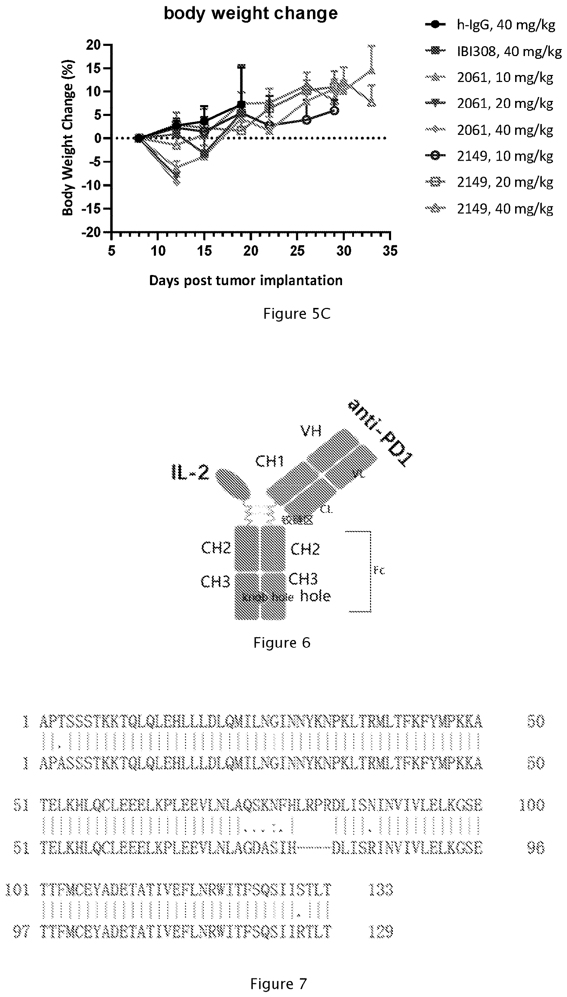

Figure 5 shows a comparison of in vivo efficacy between control molecule 2061 and immunoconjugate 2149 protein. (A) displays individual mouse tumor volume change curves reflecting the antitumor effects of 2061 and 2149 on B16F10 tumors in mice; (B) shows survival curves reflecting the antitumor effects of 2061 and 2149 on B16F10 tumors in mice; (C) shows the effects of 2061 and 2149 on mouse body weight.
Figure 6 shows a schematic diagram of the structure of αPD-1/IL2m immunoconjugate of the present invention.
Figure 7 shows a schematic diagram of a sequence alignment.

### DETAILED DESCRIPTION OF THE PREFERRED EMBODIMENTS

Before describing the invention in detail, it should be understood that the invention is not limited to the specific methods and experimental conditions described herein, as such methods and conditions may vary. Furthermore, the terminology used herein is for the purpose of describing particular embodiments only and is not intended to be limiting.

Unless otherwise defined, all technical and scientific terms used herein have the same meaning as commonly understood by one of ordinary skill in the art. For the purposes of the present invention, the following terms are defined below.

The term "about", when used in conjunction with a numerical value, is intended to cover numerical values within a range having a lower limit of 5% less than the specified numerical value and an upper limit of 5% greater than the specified numerical value.

The term "and/or", when used to connect two or more options, shall be understood to mean any one of the options or any two or more of the options.

As used herein, the term "comprising" or "including" means including the stated elements, integers, or steps, but does not exclude any other elements, integers, or steps. In this document, when the terms "comprising" or "including" are used, unless otherwise specified, they also encompass situations consisting of the stated elements, integers, or steps. For example, when referring to an antibody variable region "comprising" a specific sequence, it is also intended to encompass an antibody variable region consisting of that specific sequence.

As used herein, the term "antibody" refers to a polypeptide that at least comprises the variable regions of immunoglobulin light and heavy chains, which specifically recognize and bind to an antigen. This term covers various antibody structures, including but not limited to monoclonal antibodies, chimeric or humanized antibodies, full-length antibodies, and antibody fragments, as long as they exhibit the desired antigen-binding activity.

As used herein, a "full antibody" (interchangeably used with "full-length antibody", "complete antibody", and "intact antibody" herein) refers to a protein molecule comprising at least two heavy chains (H) and two light chains (L) interconnected by disulfide bonds. Each heavy chain consists of a heavy chain variable region (abbreviated as VH herein) and a heavy chain constant region. The heavy chain constant region is composed of three domains: CH1, CH2, and CH3. Thus, an antibody heavy chain typically comprises, in order from the N-terminus to the C-terminus: the heavy chain variable region, followed by the heavy chain constant domains CH1, CH2, and CH3. In the present context, the C-terminal region of the heavy chain, located at the C-terminus of the CH1 domain and comprising the CH2 and CH3 domains, is also referred to as the Fc region or Fc fragment. Each light chain consists of a light chain variable region (abbreviated as VL in this text) and a light chain constant region. The light chain constant region is composed of a single domain, CL. Accordingly, an antibody light chain typically comprises, in order from the N-terminus to the C-terminus: a light chain variable region and a light chain constant domain CL. The variable regions of the antibody heavy and light chains pair to form the antigen-binding domain responsible for binding to the antigen. The constant regions of the heavy and light chains do not directly participate in the binding of the antibody to the antigen but exhibit various effector functions.

As used herein, the term "anti-PD-1 half-antibody" refers to a monovalent antigen-binding polypeptide comprising one heavy chain and one light chain of a complete anti-PD-1 antibody. In such half-antibodies, typically, the VH-CH1 region of the heavy chain pairs with the VL-CL region of the light chain to form an Fab fragment that specifically binds to the PD-1 antigen, and the heavy chain Fc region is covalently linked at the C-terminus of the CH1 domain via an immunoglobulin hinge region. Therefore, the anti-PD-1 half-antibody is a polypeptide comprising an Fab fragment that specifically binds to PD-1 and is fused to the Fc region.

As used herein, the term "mutant IL-2 fusion polypeptide" refers to a fusion polypeptide comprising a mutant IL-2 polypeptide, wherein the mutant IL-2 polypeptide is fused at the C-terminus to an Fc region. Typically, the fusion polypeptide contains, from the N-terminus to the C-terminus, the mutant IL-2 polypeptide, an optional linker sequence, a hinge region, and the Fc region.

In the present context, the αPD-1/IL2m immunoconjugate refers to an immunoconjugate that comprises, consists essentially of, or consists of (i) an anti-PD-1 half-antibody and (ii) a mutant IL-2 fusion polypeptide. An exemplary structure of the αPD-1/IL2m immunoconjugate is shown in Figure 6. As illustrated in Figure 6, in the αPD-1/IL2m immunoconjugate, the VH-CH1 region of the anti-PD-1 heavy chain pairs with the VL-CL region of the light chain to form an Fab fragment that specifically binds to PD-1, and the Fc region of the mutant IL-2 fusion polypeptide pairs with the Fc region of the anti-PD-1 heavy chain and dimerizes. It should be understood that the antibody constant regions included in the immunoconjugate of the present invention can be classified into any class or subclass based on their sequences, and can be native sequence constant regions or variant sequence constant regions.

In the present context, the class and subclass of an antibody constant region (e.g., an Fc region) refer to the category determined by the constant region sequence. Generally, antibody light chain constant regions can be classified into two types (referred to as kappa (κ) and lambda (λ)) based on their amino acid sequences. Antibody heavy chain constant regions can be classified into five major different types based on their amino acid sequences: IgA, IgD, IgE, IgG, and IgM, and several of these types can be further divided into subclasses, such as IgG1, IgG2, IgG3, IgG4, IgA1, and IgA2. Therefore, when referring to an immunoconjugate having an Fc region of the IgG class, it means that the immunoconjugate has an Fc region classified as the IgG type based on its amino acid sequence. Similarly, when referring to an immunoconjugate having a kappa light chain constant region, it means that the immunoconjugate has a light chain constant region classified as the kappa type based on its amino acid sequence.

In the present context, the term "native sequence Fc region" encompasses naturally occurring Fc region sequences of various immunoglobulins, such as Fc region sequences of various Ig subtypes and their allotypes (Gestur Vidarsson et al., IgG subclasses and allotypes: from structure to effector functions, 20 October 2014, doi: 10.3389/fimmu.2014.00520.). In some embodiments, the human IgG heavy chain Fc region comprises an amino acid sequence extending from Cys226 or from Pro230 to the carboxyl terminus of the heavy chain. However, the C-terminal lysine (Lys447) of the Fc region may or may not be present. In further embodiments, the human IgG heavy chain Fc region comprises at its N-terminus a hinge sequence or a partial hinge sequence of a native immunoglobulin, such as the sequence from E216 to T225 or from D221 to T225 according to EU numbering.

As used herein, the term "variant sequence Fc region" refers to an Fc region polypeptide that comprises modifications relative to a native sequence Fc region polypeptide. The modifications may include addition, deletion, or substitution of amino acid residues. Substitutions may include naturally occurring amino acids and non-naturally occurring amino acids. The purpose of the modifications may be to alter the binding of the Fc region to its receptor and the resulting effector functions.

As used herein, the terms "complementarity determining region" or "CDR region" or "CDR" or "hypervariable region" are used interchangeably and refer to regions within the variable domain of an antibody that are highly variable in sequence and form structurally defined loops ("hypervariable loops") and/or contain antigen-contact residues ("antigen contact points"). The CDR is mainly responsible for binding to an antigenic epitope. In the present context, the CDRs of the antibody heavy chain and light chain are sequentially numbered from the N-terminus, commonly referred to as CDR1, CDR2, and CDR3. CDRs located within the variable domains of an antibody's heavy chain are also referred to as HCDR1, HCDR2, and HCDR3, and CDRs located within the variable domains of an antibody's light chain are referred to as LCDR1, LCDR2, and LCDR3. For a given amino acid sequence of a light chain variable region or heavy chain variable region, various well-known schemes in the art can be employed to determine its CDR sequences. Multiple schemes are well-known in the art for determining CDR sequences within a given VH or VL amino acid sequence, including, for example: Chothia definition (Chothia et al. (1989) Nature 342: 877-883, Al-Lazikani et al., "Standard conformations for the canonical structures of immunoglobulins", Journal of Molecular Biology, 273, 927-948 (1997)), Kabat definition (Kabat et al., Sequences of Proteins of Immunological Interest, 4th Ed., U.S. Department of Health and Human Services, National Institutes of Health (1987)), AbM definition (University of Bath), Contact definition (University College London), IMGT definition (http://imgt.cines.fr), and the North CDR definition based on affinity propagation clustering using a large number of crystal structures. Additionally, the CDR can also be determined based on the same Kabat numbered positions as the reference CDR sequence. Unless otherwise specified, in the present invention, when referring to the residue positions in the heavy/light chain variable regions of an antibody, it refers to the numbering positions according to the Kabat numbering system (Kabat et al., Sequences of Proteins of Immunological Interest, 5th Ed. Public Health Service, National Institutes of Health, Bethesda, Md. (1991)).

In the present context, "sequence identity" refers to the degree of sequence similarity on a nucleotide-by-nucleotide or amino acid-by-amino acid basis within a comparison window. The "percentage of sequence identity" can be calculated as follows: aligning the two sequences optimally within a comparison window, determining the number of positions where the same nucleic acid bases (e.g., A, T, C, G, I) or the same amino acid residues (e.g., Ala, Pro, Ser, Thr, Gly, Val, Leu, Ile, Phe, Tyr, Trp, Lys, Arg, His, Asp, Glu, Asn, Gln, Cys, and Met) are present in both sequences to obtain the number of matched positions, dividing the number of matched positions by the total number of positions in the comparison window (i.e., the window size), and multiplying the result by 100 to yield the percentage of sequence identity. Optimal alignment for determining the percentage of sequence identity can be achieved in various ways known in the art, for example, by using publicly available computer software such as BLAST, BLAST-2, ALIGN, or Megalign (DNASTAR) software. A person skilled in the art can determine suitable parameters for sequence alignment, including any algorithms required to achieve maximal alignment over the full length of the sequences being compared or within a target sequence region.

In the context of antibody sequences, the percentage of amino acid sequence identity is determined by optimally aligning the candidate antibody sequence with a given antibody sequence, preferably according to the Kabat numbering scheme. In the absence of a specified comparison window (i.e., the target antibody region to be compared), alignment will be performed over the full length of the given antibody sequence.

As used herein, the term "conservative substitution" refers to an amino acid substitution that does not adversely affect or alter the biological function of the protein/polypeptide containing the amino acid sequence. For example, conservative substitutions can be introduced by standard techniques known in the art, such as site-directed mutagenesis and PCR-mediated mutagenesis. A typical conservative amino acid substitution involves replacing one amino acid with another having similar chemical properties (e.g., charge or hydrophobicity). The table of conserved substitutions for functionally similar amino acids is well-known in the art.

As used herein, the term "immunoconjugate formulation" refers to a preparation that is in a form allowing the biological activity of the immunoconjugate as the active ingredient to be effectively exerted, and does not contain other species that would have unacceptable toxicity for the subject to whom the formulation is to be administered. Such formulations are typically sterile. Generally, the formulations contain pharmaceutically acceptable excipients. A "pharmaceutically acceptable" excipient is an agent that can be reasonably administered to a mammalian subject so that an effective dose of the active ingredient used in the formulation can be delivered to the subject. The concentration of the excipient is compatible with the mode of administration, for example, it may be acceptable for injection.

In the present context, a "stable" immunoconjugate formulation refers to a formulation in which the immunoconjugate retains an acceptable degree of physical stability and/or chemical stability after storage under specified conditions. Although the immunoconjugate contained in the immunoconjugate formulation may not maintain 100% of its chemical structure after storage for a specified period, it is generally considered "stable" if it maintains about 80%, about 85%, about 90%, about 95%, about 96%, about 97%, about 98%, or about 99% of the immunoconjugate structure or function after storage for a specified period. In some embodiments, the immunoconjugate of the present invention substantially retain their physical and chemical stability after storage. Various analytical techniques known in the art can be used to determine protein stability, see, for example, Peptide and Protein Drug Delivery, 247-301, Vincent Lee Ed., Marcel Dekker, Inc., New York, N.Y., Pubs (1991) and Jones, A. Adv. Drug Delivery Rev. 10: 29-90 (1993). Stability can be measured at selected temperatures and for selected storage durations. For example, the storage time may be selected based on the expected shelf life of the formulation. Alternatively, accelerated stability testing or high-temperature stress forced testing may be employed. In some embodiments, stability testing is conducted by subjecting the formulation to various stress tests. These tests may represent extreme conditions that the formulated product may encounter during manufacturing, storage, or transportation, or conditions that may accelerate the instability of the immunoconjugate in the formulation outside of manufacturing, storage, or transportation. For example, the formulated immunoconjugate may be filled into glass vials to assess the stability of the immunoconjugate under high-temperature stress.

If, after a period of storage, the formulation shows no aggregation, precipitation, turbidity, and/or denaturation, or shows very minimal aggregation, precipitation, turbidity, and/or denaturation, the immunoconjugate is considered to "maintain its physical stability" in the formulation. Since aggregation of the immunoconjugate in the formulation may potentially lead to an increased immune response in patients, resulting in safety concerns. Therefore, it is necessary to minimize or prevent aggregation of the immunoconjugate in the formulation. Light scattering methods may be used to detect visible aggregates in the formulation. SEC may be used to detect soluble aggregates in the formulation. In addition, the stability of the formulation can be indicated by visually inspecting the appearance, color, and/or clarity of the formulation, or by detecting the turbidity of the formulation using the OD350nm method, or by determining the purity of the formulation using the non-reduced CE-SDS method. In one embodiment, the stability of the formulation is measured by determining the percentage of immunoconjugate protein monomer in the formulation after storage at a specific temperature for a specific period of time, wherein a higher percentage of immunoconjugate protein monomer in the formulation indicates higher stability of the formulation.

As used herein, the term "immunoconjugate protein monomer" (or "immunoconjugate monomer" or "monomer") in the context of formulation stability refers to a single intact functional immunoconjugate protein molecule. The immunoconjugate protein monomer is distinct from protein aggregates composed of multiple such protein monomers, as well as from immunoconjugate protein fragments, such as half-antibody portions or IL-2 mutant fusion protein portions that are part of the immunoconjugate protein. SEC-HPLC analysis can be used to differentiate the monomeric form of the immunoconjugate protein from the aggregated form of the immunoconjugate protein. Non-reduced CE-SDS analysis can also be used to differentiate the monomeric form of the immunoconjugate protein from the fragmented form of the immunoconjugate protein.

"Acceptable degree" of physical stability may be indicated by the detection of at least about 92% immunoconjugate protein monomer in the formulation after storage at a specific temperature for a specific period of time. In some embodiments, after storage at a specific temperature for at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, or longer, an acceptable degree of physical stability is indicated by the maintenance of at least about 88%, 89%, 90%, 91%, 92%, 93%, 94%, 95%, 96%, 97%, 98%, or 99% immunoconjugate protein monomer in the formulation. When evaluating physical stability, the specific temperature for storage of the pharmaceutical formulation may be any temperature from about -80°C to about 45°C, for example, storage at about -80°C, about -30°C, about -20°C, about 0°C, about 4°C-8°C, about 5°C, about 25°C, about 35°C, about 37°C, about 40°C, about 42°C, or about 45°C. For example, if at least about 88%, 89%, 90%, 91%, 92%, 93%, 94%, 95%, 96%, 97%, 98%, or 99% immunoconjugate protein monomer is detected after storage at about 40°C±2°C for 2 weeks or 4 weeks, the pharmaceutical formulation is considered stable. If at least about 88%, 89%, 90%, 91%, 92%, 93%, 94%, 95%, 96%, 97%, 98%, or 99% immunoconjugate protein monomer is detected after storage at about 25°C±2°C for 4 weeks, 2 months, or 3 months, the pharmaceutical formulation is considered stable. If at least about 88%, 89%, 90%, 91%, 92%, 93%, 94%, 95%, 96%, 97%, 98%, or 99% of the immunoconjugate protein monomer is detected after storage at about 5°C ±3°C for 3 months, the pharmaceutical formulation is considered stable.

After a period of storage, if the immunoconjugate protein in the formulation does not exhibit significant chemical alterations, the immunoconjugate protein can be considered to "maintain its chemical stability" in the formulation. Most chemical instabilities arise from the formation of covalent modifications of the immunoconjugate protein (e.g., charge heterogeneity of the immunoconjugate protein). For example, basic heterogeneity can be formed by aspartic acid isomerization, N- and C-terminal modifications, while acidic heterogeneity can be generated by deamidation, sialylation, and glycation. Chemical stability can be assessed by detecting and/or quantifying the chemically altered forms of the immunoconjugate protein. For instance, charge heterogeneity of the immunoconjugate protein in the formulation can be detected by imaging capillary isoelectric focusing (iCIEF). In one embodiment, the stability of the formulation is measured by determining the percentage change in charge heterogeneity of the immunoconjugate protein in the formulation after storage at a specific temperature for a specific period, wherein a smaller change value indicates higher stability of the formulation.

An "acceptable degree" of chemical stability may be indicated by a percentage change in charge heterogeneity of the immunoconjugate protein (e.g., main species, acidic species, or basic species) in the formulation after storage at a specific temperature for a specific period not exceeding 50%, such as not exceeding 30%, or not exceeding 20%. In some embodiments, after storage at a specific temperature for at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, or longer, an acceptable degree of chemical stability may be manifested as a percentage change in charge heterogeneity of the main species not exceeding about 50%, 40%, 30%, 20%, or 15%. When evaluating chemical stability, the temperature for storing the pharmaceutical formulation can be any temperature from about -80°C to about 45°C, such as storage at about -80°C, about -30°C, about -20°C, about 0°C, about 4°C-8°C, about 5°C, about 25°C, or about 45°C. For example, if after storage at 5°C for 24 months, the percentage change in charge heterogeneity of the main species is less than about 25%, 24%, 23%, 22%, 21%, 20%, 19%, 18%, 17%, 16%, 15%, 14%, 13%, 12%, 10%, 9%, 8%, 7%, 6%, 5%, 4%, 3%, 2%, 1%, 0.5%, or 0.1%, the pharmaceutical formulation may be considered stable. If after storage at 25°C for 2 months, the percentage change in charge heterogeneity of the main species is less than about 20%, 19%, 18%, 17%, 16%, 15%, 14%, 13%, 12%, 10%, 9%, 8%, 7%, 6%, 5%, 4%, 3%, 2%, 1%, 0.5%, or 0.1%, the pharmaceutical formulation may also be considered stable. If after storage at 40°C for 1 month, the percentage change in charge heterogeneity of the main species is less than about 50%, 40%, 30%, 20%, 10%, 5%, or 4%, the pharmaceutical formulation may also be considered stable.

The term "lyophilized formulation" refers to a composition obtained or obtainable by freeze-drying a liquid formulation. Preferably, it is a solid composition with less than 5%, preferably less than 3% water content.

The term "reconstituted formulation" refers to a liquid formulation obtained by dissolving and/or suspending a solid formulation (e.g., a lyophilized formulation) in a physiologically acceptable solution.

The term "room temperature" as used herein refers to a temperature of 15°C to 30°C, preferably 20°C to 27°C, more preferably 25°C.

"Stress conditions" refer to an environment that is chemically and/or physically unfavorable for the immunoconjugate, which may lead to unacceptable destabilization of the immunoconjugate protein.

"High-temperature stress" refers to storing the immunoconjugate formulation at room temperature or even higher temperatures (e.g., 40°C±2°C) for a period of time. The stability of the immunoconjugate formulation can be examined through room temperature accelerated testing or high-temperature stress forced testing.

As used herein, the term "parenteral administration" refers to modes of administration other than enteral and topical administration, generally by injection or infusion, and includes, but is not limited to, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural, and sternal injection and infusion. In some embodiments, the immunoconjugate formulation of the present invention is administered parenterally to a subject. In one embodiment, the immunoconjugate formulation of the present invention is administered to a subject by subcutaneous, intradermal, intramuscular, or intravenous injection.

### I. The immunoconjugate formulation of the present invention

Herein, the formulation of the αPD-1/IL2m immunoconjugate according to the present invention, also referred to as "the immunoconjugate formulation of the present invention" or "the formulation of the present invention", means a formulation comprising the αPD-1/IL2m immunoconjugate according to the present invention as an active ingredient and comprising pharmaceutically acceptable excipients. The formulation of the present invention not only enables the αPD-1/IL2m immunoconjugate as the active ingredient to be formulated in a manner suitable for administration to a subject but also maintains its stability during storage and subsequent use.

Accordingly, in a first aspect, the present invention provides a liquid formulation comprising the αPD-1/IL2m immunoconjugate, which comprises:
(i) an αPD-1/IL2m immunoconjugate;
(ii) a buffering agent;
(iii) a stabilizer; and
(iv) Surfactant.

The formulation of the present invention can be prepared as a liquid formulation in aqueous form for use in, for example, ready-to-use prefilled syringes, or can be prepared as a lyophilized formulation for reconstitution (i.e., redissolution) immediately before use by dissolving and/or suspending in a physiologically acceptable solution. In some embodiments, the formulation of the present invention is in the form of a liquid formulation.

### Properties of the liquid formulation of the present invention

The liquid formulation of the present invention can be stably stored for a long period, for example, at least 24 months or longer. In one embodiment, the liquid formulation of the present invention can be stored under conditions ranging from about -80°C to about 45°C, for example, -80°C, about -30°C, about -20°C, about 0°C, about 5°C, about 25°C, about 35°C, about 38°C, about 40°C, about 42°C, or about 45°C, for at least 5 days, at least 10 days, at least 20 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, at least 36 months, or longer, and remains stable.

In some embodiments, the liquid formulation of the present invention remains stable at about 2°C-8°C for at least 3 months, at least 12 months, or at least 24 months. In some embodiments, the liquid formulation of the present invention remains stable at room temperature or, for example, about 25°C, for at least 1 month, at least 2 months, or at least 3 months. In some embodiments, the liquid formulation of the present invention remains stable at about 40°C for at least 1 week, at least 2 weeks, at least 3 weeks, or at least 1 month.

In one embodiment, the stability of the formulation after storage can be indicated by detecting changes in the appearance, visible particulates, protein content, pH, purity, charge heterogeneity, and/or polysorbate surfactant content of the formulation. In one embodiment, the stability of the liquid formulation of the present invention can be tested in forced degradation studies under high-temperature stress, for example after storage at 40°C±2°C for at least 1 week, 2 weeks, or 1 month, or in accelerated studies, for example after storage at 25°C±2°C for at least 1 month, 2 months, or 3 months, or in long-term studies, for example after storage at 5°C±3°C for at least 2 months or 3 months.

In one embodiment, the stability of the liquid formulation of the present invention is visually inspected after storage, wherein the liquid formulation of the present invention remains clear to slightly opalescent in appearance, colorless to pale yellow, and free from foreign particles. In one embodiment, no visible foreign particles are observed in the formulation upon visual inspection under a clarity tester. In one embodiment, the stability of the liquid formulation of the present invention is checked by measuring the change in protein content after storage, wherein, for example, by UV spectrophotometry, the change rate in protein content relative to the initial value on Day 0 of storage does not exceed 20%, preferably does not exceed 10%, for example 7-8%, more preferably does not exceed 5%. In one embodiment, the stability of the liquid formulation of the present invention is checked by measuring the change in pH after storage, wherein the change relative to the initial value on Day 0 of storage does not exceed 0.3, preferably does not exceed 0.2, more preferably does not exceed 0.1. In one embodiment, the stability of the liquid formulation of the present invention is checked by measuring the change in purity after storage, wherein, by size-exclusion high-performance liquid chromatography (SEC-HPLC), the change in monomer purity of the immunoconjugate relative to the initial value on Day 0 of storage does not exceed 10%, for example does not exceed 5%, 4%, 3%, or for example does not exceed 1-2%, preferably does not exceed 1%. In one embodiment, the stability of the liquid formulation of the present invention is checked by measuring the change in purity after storage, wherein, by non-reduced sodium dodecyl sulfate capillary electrophoresis (nrCE-SDS), the decrease in monomer purity of the immunoconjugate does not exceed 10%, for example does not exceed 5%, 4%, 3%, preferably does not exceed 2%. In one embodiment, the stability of the liquid formulation of the present invention is tested by imaging capillary isoelectric focusing (iCIEF) after storage, wherein the total change in charge heterogeneity (main species, acidic species, and basic species) of the immunoconjugate relative to the initial value on Day 0 of storage does not exceed 50%, for example does not exceed 40%, 30%, 20%, 10%, 5%. In one embodiment, the stability of the liquid formulation of the present invention is tested by high-performance liquid chromatography with fluorescence detection (HPLC-FLD) after storage, wherein the change in polysorbate surfactant content relative to the initial value on Day 0 of storage does not exceed 0.3 mg/mL, for example does not exceed 0.2 mg/mL, or does not exceed 0.1 mg/mL. Preferably, after storage, the polysorbate 80 surfactant content in the formulation is between 0.1 mg/mL and 0.5 mg/mL, for example between 0.2 mg/mL and 0.4 mg/mL, preferably about 0.3 mg/mL.

In one embodiment, the formulation is stable after storage, for example after storage at 2-8°C for at least 24 months, or at room temperature for at least 3 months, or at 40°C±2°C for 1 month, preferably having one or more of the following characteristics:
(i) As measured by SEC-HPLC, the formulation has a purity greater than 90%, preferably greater than 95%, 96%, or 97%;
(ii) As measured by non-reduced CE-SDS, the formulation has a purity greater than 90%, preferably greater than 92%, 94%, 96%, or 98%;
(iii) As measured by iCIEF, the total change in charge heterogeneity of the immunoconjugate (main species, acidic species, and basic species) in the formulation relative to the initial value on storage Day 0 does not exceed 50%, for example, does not exceed 40%, 30%, 20%, 10%, or 5%;
(iv) As measured by HPLC-FLD, the change in polysorbate 80 content in the formulation relative to the initial value on storage Day 0 does not exceed 0.3 mg/mL, for example, does not exceed 0.2 mg/mL, 0.10 mg/mL, or 0.05 mg/mL; and
(v) As measured by ELISA, the decrease in relative binding activity of the immunoconjugate to IL-2 receptor and PD-1 in the formulation relative to the initial value on storage Day 0 does not exceed 30%, for example, is 80%-110%, such as about 70%, about 80%, about 90%, about 100%, or about 110%. The SEC-HPLC, non-reduced CE-SDS, iCIEF, HPLC-FLD, and ELISA methods for determining the stability of the formulation can be performed in a manner known in the art or as described in the examples.

The liquid formulation of the present invention can remain stable under shaking and freeze-thaw conditions. In one embodiment, the stability of the formulation under shaking and freeze-thaw conditions can be indicated by detecting changes in appearance, visible particulates, protein content, pH, purity, charge heterogeneity, and/or polysorbate surfactant content. In one embodiment, the stability of the liquid formulation of the present invention can be tested after shaking, for example, at 650 rpm at room temperature in the dark for 1 day, 2 days, or 3 days. In one embodiment, the stability of the liquid formulation of the present invention can be tested after freeze-thaw cycles, for example, after 1, 2, 3, 4, 5, or 6 freeze-thaw cycles consisting of freezing at -20°C and thawing at 5°C±3°C.

Room temperature accelerated testing, high temperature stress testing, and long-term testing for examining the stability properties of the formulation of the present invention may be conducted in a manner known in the art or as described in the examples.

### Components of the liquid formulation of the present invention

### (i) αPD-1/IL2m immunoconjugate

The αPD-1/IL2m immunoconjugate comprising in the formulation of the present invention comprises (i) an anti-PD-1 half-antibody and (ii) a mutant IL-2 fusion polypeptide. The anti-PD-1 half-antibody according to the present invention comprises an Fab fragment that specifically binds to PD-1 and is fused to an Fc region, and the mutant IL-2 fusion polypeptide according to the present invention comprises a mutant IL-2 polypeptide fused to an Fc region. The mutant IL-2 polypeptide and the αPD-1/IL2m immunoconjugate suitable for the present invention are disclosed in PCT/CN2022/120265, which is incorporated herein by reference in its entirety.

The following provides a detailed description of the components of the αPD-1/IL2m immunoconjugate of the present invention, the anti-PD-1 half-antibody and the mutant IL-2 fusion polypeptide, as well as exemplary αPD-1/IL2m immunoconjugates. It will be understood by those skilled in the art that, unless the context clearly indicates otherwise, any combination of any technical features described in these sections is contemplated by the present invention, and the αPD-1/IL2m immunoconjugate of the present invention may comprise any such combination of features.

### Mutant IL-2 polypeptide

The mutant IL-2 fusion polypeptide according to the present invention comprises a mutant IL-2 polypeptide fused to an Fc region. In some embodiments, the mutant IL-2 polypeptide according to the present invention, compared to wild-type IL-2, comprises mutations that weaken the binding affinity for the IL-2Rβγ receptor at the interface where IL-2 binds to IL-2Rβγ; and has a shortened B'C' loop that improves protein expression and/or purity.

As used herein, the wild-type "Interleukin-2" or "IL-2" refers to the parental IL-2 protein serving as the template for introducing the mutations or combinations of mutations of the present invention, preferably a naturally occurring IL-2 protein, such as a natural IL-2 protein derived from humans or non-human primates, including unprocessed (e.g., with signal peptide) and processed (e.g., without signal peptide) forms. A full-length natural human IL-2 sequence comprising a signal peptide is shown in SEQ ID NO: 1, and the sequence of its mature protein is shown in SEQ ID NO: 2. This description also includes variants of natural IL-2, for example, a natural human IL-2 protein (UniProt: P60568) with a C125S mutation introduced at position 125 falls within the scope of wild-type IL-2 of the present invention. An example of a wild-type human IL-2 protein comprising the C125S mutation is shown in SEQ ID NO: 3. Preferably, the wild-type IL-2 sequence has at least 85%, 95%, or even at least 96%, 97%, 98%, or 99% or higher amino acid sequence identity to the amino acid sequence of SEQ ID NO: 1, 2, or 3.

As used herein, when referring to amino acid positions in IL-2 polypeptides or IL-2 sequence segments, they are determined by reference to the amino acid sequence of wild-type human IL-2 protein (also referred to as IL-2^{WT}) SEQ ID NO: 3. Corresponding amino acid positions in other IL-2 proteins or polypeptides (including full-length sequences or truncated fragments) can be identified by aligning their amino acid sequences with SEQ ID NO: 3. Therefore, in the present invention, unless otherwise specified, the amino acid positions of IL-2 proteins or polypeptides are numbered according to SEQ ID NO: 3. For example, when referring to "F42", it means the phenylalanine residue F at position 42 of SEQ ID NO: 3 or the corresponding amino acid residue at the aligned position in other IL-2 polypeptide sequences. Additionally, for clarity, when the mutant IL-2 of the present invention involves truncation or deletion of certain specific segments (e.g., the sequence of the B'C' loop, i.e., the 11 amino acid residues at positions 73-83 of SEQ ID NO: 3), if necessary, the region can be visually inspected and gaps introduced to ensure that, when the mutant IL-2 is aligned with SEQ ID NO: 3, the truncated or deleted segment with gaps corresponds to the respective segment in SEQ ID NO: 3 in terms of position and amino acid residue numbering. For example, for a mutant IL-2 polypeptide (SEQ ID NO: 4) comprising a B'C' loop sequence truncated to 7 amino acid residues (AGDASIH), 4 gaps can be introduced into the truncated B'C' loop by visual inspection, such that when the mutant IL-2 polypeptide is aligned with SEQ ID NO: 3, the truncated B'C' loop with introduced gaps corresponds to the B'C' loop sequence of SEQ ID NO: 3 in the respective positional segment and has amino acid residue numbering at positions 73-83, as shown in Figure 7.

For sequence alignment to determine amino acid positions, the Basic Local Alignment Search Tool available at https://blast.ncbi.nlm.nih.gov/Blast.cgi can be used with default parameters.

In the present context, amino acid mutations in the IL-2 mutant can be amino acid substitutions, deletions, insertions, and/or additions. Any combination of substitutions, deletions, insertions, and additions can be performed to obtain a final mutant protein construct with desired properties (e.g., reduced IL-2Rα binding affinity and/or improved druggability and/or weakened IL-2Rβγ). For example, the alanine residue at position 1 of full-length human IL-2 can be deleted, or one or several amino acids in the B'C' loop can be deleted to shorten the length of the loop. Alternatively, the entire or partial B'C' loop sequence of wild-type IL-2 can be replaced with a different sequence (e.g., the B'C' loop of IL-15) to obtain a shortened B'C' loop sequence.

In the present context, when referring to IL-2 mutant proteins, single amino acid substitutions are described as follows: [original amino acid residue/position/substituted amino acid residue]. For example, the substitution of lysine at position 35 with glutamic acid can be denoted as K35E. When multiple alternative amino acid substitutions (e.g., D, E) are possible at a given position (e.g., K35), the amino acid substitution can be represented as: K35D/E. Correspondingly, individual amino acid substitutions can be connected by a plus sign "+" or a minus sign "-" to indicate combined mutations at multiple specified positions. For example, the combined mutations at positions F42A, N88R, and S127E can be represented as: F42A+N88R+S127E, or F42A-N88R-S127E.

In the present context, the sequence differences between the IL-2 mutant polypeptide according to the present invention and the wild-type IL-2 polypeptide can be described by sequence identity or expressed by the number of differing amino acids between them. In one embodiment, the IL-2 mutant polypeptide according to the present invention has at least 85%, 86%, 87%, 88%, 89% identity with the wild-type IL-2 polypeptide, preferably more than 90% identity, such as at least 95%, 96%, or 97% identity. In other embodiments, apart from the specifically defined mutations according to the present invention (IL-2Rα binding interface mutations, IL-2βγ binding interface and/or shortened B'C' loop), the IL-2 mutant polypeptide may also have no more than 15 mutations, such as 1-10 mutations, or 1-5 mutations, for example, 0, 1, 2, 3, or 4 mutations, compared to the wild-type polypeptide. In one embodiment, the remaining mutations may be conservative substitutions.

In the present context, the terms "B'C' Loop", "B'C' loop region", or "B'C' loop sequence" are used interchangeably and refer to the connecting sequence between the B helix and C helix of the IL-2 protein. The B'C' loop sequence of an IL-2 protein can be determined through crystal structure analysis of IL-2 (e.g., PDB:2ERJ). For the purposes of the present invention, according to the numbering of SEQ ID NO:3, the B'C' loop sequence refers to the sequence connecting the residue at position 72 and the residue at position 84 in the IL-2 polypeptide. In the wild-type IL-2 proteins of SEQ ID NO: 1, 2, and 3, this connecting sequence comprises 11 amino acids from A73 to R83. Accordingly, as used herein, the terms "shortened loop region" or "shortened B'C' loop region" refer to a mutant protein having a B'C' loop sequence with reduced length compared to the wild-type IL-2 protein, i.e., the length of the connecting sequence between amino acid residues aa72 and aa84 is shortened according to the numbering of SEQ ID NO: 3. The "shortened loop region" may be achieved by substitution or truncation of the loop sequence. The substitution or truncation may occur in any region or portion of the B'C' loop sequence. For example, the substitution or truncation may involve replacement of the loop sequence A73-R83 (e.g., replacement with the IL-15 B'C loop region) or truncation of one or more amino acid residues from the C-terminus of this sequence to obtain a B'C' loop sequence with a length of less than 10, 9, 8, 7, 6, or 5 amino acids. As another example, the substitution or truncation may involve replacement of the loop sequence Q74-R83 or truncation of one or more amino acid residues from the C-terminus of this sequence. After performing the substitution or truncation, if necessary, single amino acid substitutions may be further introduced into the loop sequence, such as amino acid substitutions to eliminate glycosylation, and/or revertant mutations to further improve the properties of the mutant protein, such as druggability. Therefore, in the present context, the mutant shortened B'C' loop can be described by the sequence between residue at position 72 and residue at position 84 after introducing the mutation.

In the present context, "IL-2Rα binding interface" mutation refers to mutations occurring at amino acid sites where IL-2 interacts with IL-2Rα (i.e., CD25). These interaction sites can be determined by analyzing the crystal structure of IL-2 in complex with its receptor (e.g., PDB:1Z92). In some embodiments, the mutation particularly refers to mutations in the amino acid residues 35-72 region of IL-2. Preferably, compared to the corresponding protein before introducing the mutation, the IL-2 protein comprising said mutation has reduced or eliminated IL-2Rα binding.

In the present context, "IL-2βγ binding interface" mutation refers to mutations occurring at amino acid sites where IL-2 interacts with IL-2Rβγ (i.e., CD122 and CD132). These interaction amino acid sites can be determined by crystal structure analysis of IL-2 in complex with its receptor (e.g., PDB:2ERJ). In some embodiments, the mutation particularly refers to mutations in the amino acid residues 12-20, amino acid residues 84-95, and amino acid residues 126-130 regions of IL-2. Preferably, compared to the corresponding protein before introducing the mutation, the IL-2 protein comprising said mutation has weakened IL-2Rβγ binding.

In some embodiments, the mutant IL-2 polypeptide according to the present invention, compared to wild-type IL-2 (preferably human IL-2, more preferably IL-2 comprising the sequence of SEQ ID NO: 3) comprising the following mutations:
(i) At the IL-2/IL-2Rβγ binding interface, particularly at least one position selected from positions 88, 127, and/or 130, having mutations that weaken binding to the IL-2Rβγ receptor;
   And
(ii) A shortened B'C' loop region (i.e., the sequence connecting amino acid residues aa72 and aa84), preferably, the shortened loop region has a length of less than 10, 9, 8, 7, 6, or 5 amino acids, and more preferably 7 amino acids in length; preferably, the shortened B'C' loop region results in improved protein expression levels and/or purity,

Wherein the amino acid positions are numbered according to SEQ ID NO: 3.

Preferably, the IL-2Rβγ binding interface mutation comprises one or more mutations selected from the following or combinations thereof:
N88D, N88R, S127E, S130R, N88R+S130R, N88R+S127E.

Preferably, the shortened B'C' loop region has the sequence A(Q/G)SKN(F/I)H, or more preferably, has a B'C' loop sequence (i.e., the sequence connecting aa72 to aa84) selected from: AQSKNFH; AGSKNFH; AQSANFH; and AQSANIH.

Optionally, the mutant IL-2 polypeptide of the present invention may further comprise mutations at the IL-2/IL-2Rα binding interface, particularly at positions 35 and/or 42, that eliminate or reduce binding affinity to the IL-2Rα receptor. Preferably, the IL-2Rα binding interface mutation comprises mutations K35E and/or F42A. In some embodiments, the IL-2 mutant polypeptide according to the present invention, relative to wild-type IL-2, comprises:
(i) N88R + S130R;
   N88D;
   N88R;
   F42A+N88R + S127E; or
   K35E+N88R + S127E; and
(ii) B'C' loop sequence AGDASIH or AQSKNFH;
   and optionally (iii) T3A.

In a preferred embodiment, the mutant IL-2 polypeptide comprises: (i) T3A +N88R + S130R; and (ii) B'C' loop sequence AGDASIH. Preferably, the mutant IL-2 comprises the amino acid sequence of SEQ ID NO: 4 or has at least 85%, 90%, 91%, 92%, 93%, 94%, 96%, or 96% sequence identity thereto.

### Anti-PD-1 half-antibody

In one embodiment, the anti-PD-1 half-antibody according to the present invention comprises: three heavy chain CDR sequences (i.e., HCDR1, HCDR2, and HCDR3) contained in the heavy chain variable region sequence of SEQ ID NO: 8; and three light chain CDR sequences (i.e., LCDR1, LCDR2, and LCDR3) contained in the light chain variable region sequence of SEQ ID NO: 15. In some embodiments, the CDRs are defined according to Chothia, AbM, Kabat, IMGT, or any combination thereof, for example, according to Kabat or Chothia or a combination thereof. In some embodiments, the CDRs are CDR sequences defined according to the North scheme. In a preferred embodiment, the light chain CDRs (LCDRs) are CDR sequences defined according to the Kabat scheme; the heavy chain CDRs (HCDRs) are CDR sequences defined according to a combination of the Kabat and Chothia schemes.

In one embodiment, the anti-PD-1 half-antibody according to the present invention comprises:
- HCDR1, HCDR2, and HCDR3, each comprising or consisting of the amino acid sequences shown in SEQ ID NO: 9, SEQ ID NO: 10, and SEQ ID NO: 11, respectively; and
- LCDR1, LCDR2, and LCDR3, each comprising or consisting of the amino acid sequences shown in SEQ ID NO: 16, SEQ ID NO: 17, and SEQ ID NO: 18, respectively.

In one embodiment, the anti-PD-1 half-antibody comprises: a heavy chain variable region sequence of SEQ ID NO: 8 or a sequence having at least 90%, 91%, 92%, 93%, 94%, 96%, or 96% sequence identity thereto; and a light chain variable region sequence of SEQ ID NO: 15 or a sequence having at least 90%, 91%, 92%, 93%, 94%, 96%, or 96% sequence identity thereto. Preferably, the anti-PD-1 half-antibody comprises: a heavy chain variable region of SEQ ID NO: 8 and a light chain variable region of SEQ ID NO: 15.

### Fc region of the αPD-1/IL2m immunoconjugate

The αPD-1/IL2m immunoconjugate according to the present invention comprises (i) an anti-PD-1 half-antibody and (ii) a mutant IL-2 fusion polypeptide. Preferably, the anti-PD-1 half-antibody and the mutant IL-2 fusion polypeptide correctly pair through their respective Fc regions and the hinge region located at the N-terminus of the Fc region to form the immunoconjugate according to the present invention.

In one embodiment, the Fc regions of the mutant IL-2 fusion polypeptide and the anti-PD-1 heavy chain may be Fc regions of the IgG class, or Fc regions of the IgG1, IgG2, IgG3, or IgG4 subclass. In one embodiment, the Fc region comprises knob-in-hole mutations to promote correct pairing of the anti-PD-1 half-antibody and the mutant IL-2 fusion polypeptide. In one embodiment, the Fc region of the mutant IL-2 fusion polypeptide comprises a knob mutation, and the Fc region of the anti-PD-1 heavy chain comprises a hole mutation. In one embodiment, the knob mutation comprises mutations T366W and S354C (EU numbering), and the hole mutation comprises mutations T366S, L368A, Y407V, and Y349C (EU numbering).

### Exemplary αPD-1/IL2m immunoconjugate

In some preferred embodiments, the αPD-1/IL2m immunoconjugate according to the present invention comprises or consists of (i) a mutant IL-2 fusion polypeptide, (ii) an anti-PD-1 heavy chain, and (iii) an anti-PD-1 light chain, wherein:
- the fusion polypeptide of mutant IL-2 comprises the amino acid sequence of SEQ ID NO: 7 or a sequence having at least 90%, 91%, 92%, 93%, 94%, 96%, or 96% sequence identity thereto;
- the anti-PD-1 heavy chain comprises the amino acid sequence of SEQ ID NO: 14 or a sequence having at least 90%, 91%, 92%, 93%, 94%, 96%, or 96% sequence identity thereto; and
- the anti-PD-1 light chain comprises the amino acid sequence of SEQ ID NO: 20 or a sequence having at least 90%, 91%, 92%, 93%, 94%, 96%, or 96% sequence identity thereto.

In some more preferred embodiments, the αPD-1/IL2m immunoconjugate according to the present invention comprises or consists of a mutant IL-2 fusion polypeptide having the amino acid sequence of SEQ ID NO: 7, an anti-PD-1 heavy chain having the amino acid sequence of SEQ ID NO: 14, and an anti-PD-1 light chain having the amino acid sequence of SEQ ID NO: 20.

In some embodiments, the αPD-1/IL2m immunoconjugate of the present invention for use in the formulation of the present invention is recombinantly expressed and purified from HEK293 cells (including HEK293 cells and cells derived from HEK293 cells, such as HEK293T, HEK293F, and HEK293E cells); or from CHO cells (including CHO cells and cells derived from CHO cells, such as CHO-S, CHO-dhfr-, CHO/DG44, and ExpiCHO cells). The αPD-1/IL2m immunoconjugate of the present invention may be recombinantly expressed using methods well known in the art for recombinant expression of immunoconjugate proteins or using the methods described in the examples. Techniques for purifying therapeutic proteins to pharmaceutical grade are well known in the art, including but not limited to, Protein A capture, ion exchange chromatography, viral inactivation, viral filtration, ultrafiltration/diafiltration, and combinations thereof. Such purification techniques may be used to provide immunoconjugate drug substances with sufficient reproducibility and moderate purity for formulation preparation. As shown in the examples, the immunoconjugate used in the liquid formulation of the present invention exhibits significant anti-tumor activity while having reduced side effects.

The amount of the αPD-1/IL2m immunoconjugate comprised in the antibody formulation of the present invention may vary depending on the specific intended properties of the formulation, the specific circumstances, and the specific purpose for which the formulation is used. In some embodiments, the formulation of the present invention is a liquid formulation, which may contain about 0.5-150 mg/mL, preferably about 0.5 mg/mL to about 100 mg/mL, for example about 0.5 mg/mL to about 50 mg/mL or about 1 mg/mL to about 20 mg/mL, such as about 0.5 mg/mL, about 1 mg/mL, about 2 mg/mL, about 3 mg/mL, about 4 mg/mL, about 5 mg/mL, about 6 mg/mL, about 7 mg/mL, about 8 mg/mL, about 9 mg/mL, about 10 mg/mL, about 11 mg/mL, about 12 mg/mL, about 13 mg/mL, about 14 mg/mL, about 15 mg/mL, about 16 mg/mL, about 17 mg/mL, about 18 mg/mL, about 19 mg/mL, or about 20 mg/mL of the αPD-1/IL2m immunoconjugate of the present invention.

### (ii) Buffer

A buffer or buffer solution is typically a solution composed of a buffer pair with buffering effect, such as a "weak acid and its conjugate base" or a "weak base and its conjugate acid", for example, a mixed solution of a weak acid (e.g., acetic acid) and its salt (e.g., sodium acetate), or a mixed solution of a weak base (e.g., histidine) and its salt (e.g., histidine hydrochloride or histidine acetate).

In the present context, a buffering agent refers to a substance that can maintain the pH of a solution within an acceptable range. In some cases, the buffering agent may be a weak acid or weak base compound that constitutes a buffer or buffer solution, which can achieve the desired buffering effect when mixed with an appropriate amount of its corresponding "conjugate acid/base" (the amount depending on the predetermined desired pH), or when a strong acid or strong base is added to provide the required amount of the corresponding "conjugate acid/base" in situ. In other cases, the buffering agent may be a buffer system comprising a weak acid and its salt or a weak base and its salt. For example, for a histidine buffer, it can be prepared by titrating histidine (solid) with an acid (e.g., hydrochloric acid or acetic acid); it can also be prepared with an appropriate amount of histidine and its salt (e.g., hydrochloride or acetate) to provide the desired pH. Therefore, in the present context, when it is mentioned that the formulation of the present invention comprises a specific weak acid or weak base buffering agent, it should be understood that this also covers the case where the formulation comprises a buffer system composed of the weak acid and its salt or the weak base and its salt. For example, when referring to a formulation comprising histidine or a histidine buffering agent, it is understood that this also encompasses the situation where the formulation comprises a buffer system composed of histidine and its salts (e.g., but not limited to, hydrochloride or acetate).

In the present context, when referring to a liquid formulation comprising a certain concentration of a buffering agent, it means the combined amount of the weak acid or weak base present in the liquid formulation as the buffering agent and its corresponding conjugate acid/conjugate base. For histidine buffer, a person skilled in the art can readily calculate this concentration based on the amount of histidine and/or its salts used to prepare the formulation.

In some embodiments, the buffering agent used in the formulation of the present invention can control the pH of the formulation within a range of about 5.0 to about 6.5, for example, about 5.0 to about 5.5, preferably about 5.0. In some specific embodiments, the formulation of the present invention has a pH of about 5.0, about 5.2, about 5.4, about 5.6, about 5.8, about 6.0, about 6.2, or about 6.4. In some specific embodiments, the formulation of the present invention has a pH of 5.0±0.2.

In one embodiment, the buffering agent used in the formulation of the present invention may be selected from the group consisting of: histidine buffering agent, glutamate buffering agent, phosphate buffering agent, acetate buffering agent, citrate buffering agent, tris(hydroxymethyl)aminomethane (tris) buffering agent, and combinations thereof. In some embodiments, the (total) concentration of the buffering agent in the formulation of the present invention is about 0.5 mM to about 200 mM, about 5 mM to about 50 mM, or about 5 mM to about 20 mM.

In some embodiments, the buffering agent used in the formulation of the present invention is a histidine buffering agent or a histidine buffer system comprising histidine and its salts, particularly a histidine buffer system comprising histidine and histidine hydrochloride. In some embodiments, the liquid formulation comprises about 5 mM to about 50 mM of histidine. In one embodiment, the liquid formulation comprises from about 10 mM to about 30 mM, for example, about 10 mM, about 15 mM, about 20 mM, about 25 mM, or about 30 mM of histidine. In one embodiment, the liquid formulation of the present invention comprises about 10 mM of histidine. In yet another embodiment, the liquid formulation of the present invention comprises a combination of about 0.75 mM of histidine and about 9.25 mM of histidine hydrochloride, or a combination of about 0.12 mg/mL histidine and about 1.94 mg/mL histidine hydrochloride.

### (iii) Stabilizer

Suitable stabilizers for use in the present invention may be selected from sugars, amino acids, and combinations thereof. Furthermore, the stabilizer of the present invention may further comprise a chelating agent, such as EDTA.

The sugar as a stabilizer may be a disaccharide, trisaccharide, or polysaccharide, and the sugar may be selected from, but not limited to: sucrose, dextrose, lactose, maltose, trehalose, cyclodextrin, maltodextrin, and dextran. In one embodiment, the sugar as a stabilizer is sucrose and/or trehalose.

In some embodiments, the sugar as a stabilizer is present in the liquid formulation of the present invention at a concentration of about 50 mM to about 500 mM, preferably about 100 mM to about 400 mM, for example, about 100 mM, about 150 mM, about 200 mM, about 250 mM, about 300 mM, about 350 mM, or about 400 mM. In some embodiments, the sugar as a stabilizer is sucrose, preferably at a concentration in the liquid formulation of the present invention of about 30 mg/mL to about 120 mg/mL, for example, about 50 mg/mL, about 60 mg/mL, about 70 mg/mL, about 80 mg/mL, about 90 mg/mL, about 100 mg/mL, or about 120 mg/mL. In some embodiments, the content of the sugar (e.g., sucrose) may be further adjusted in consideration of the osmotic pressure of the liquid formulation.

In some embodiments, in addition to the sugar, the formulation of the present invention further comprises an amino acid as a stabilizer. The amino acid as a stabilizer may be selected from, but not limited to: homocysteine, cysteine, cystathionine, methionine, and arginine, preferably, the amino acid is methionine. In such cases, the sugar as a stabilizer is present at a concentration of about 50 mM to about 500 mM, preferably about 100 mM to about 400 mM, for example, about 100 mM, about 150 mM, about 200 mM, about 250 mM, about 300 mM, about 350 mM, or about 400 mM, and the amino acid as a stabilizer is present at a concentration of about 10 mM to about 200 mM, preferably about 30 mM to about 100 mM, for example, about 40 mM, about 50 mM, about 60 mM, about 70 mM, about 80 mM, about 90 mM, or about 100 mM.

In one embodiment, the liquid formulation of the present invention comprises sucrose as a stabilizer. The concentration of sucrose in the liquid formulation is about 50 mg/mL to about 120 mg/mL, for example, about 50 mg/mL, about 60 mg/mL, about 70 mg/mL, about 80 mg/mL, about 90 mg/mL, about 100 mg/mL, or about 120 mg/mL, preferably about 80 mg/mL.

In one embodiment, the liquid formulation of the present invention comprises sucrose and methionine as stabilizers. The concentration of sucrose in the liquid formulation is about 30 mg/mL to about 100 mg/mL, for example, about 50 mg/mL, about 60 mg/mL, about 70 mg/mL, or about 80 mg/mL. The concentration of methionine in the liquid formulation is about 20 mM to about 80 mM, for example, about 30 mM, about 40 mM, about 50 mM, about 60 mM. Preferably, the concentration of sucrose is about 50 mg/mL to about 80 mg/mL, and the concentration of methionine is about 30 mM to about 60 mM. More preferably, the concentration of sucrose is about 70 mg/mL, and the concentration of methionine is about 50 mM. In a preferred embodiment, the liquid formulation of the present invention comprises about 70 mg/mL sucrose and about 7.49 mg/mL methionine.

### (iv) Surfactant

As used herein, the term "surfactant" refers to an organic substance with an amphiphilic structure; that is, they consist of groups with opposing solubility tendencies, typically an oil-soluble hydrocarbon chain and a water-soluble ionic group.

In one embodiment, the surfactant in the liquid formulation of the present invention is a non-ionic surfactant, for example, alkyl poly(ethylene oxide). Specific non-ionic surfactants that may be included in the formulation of the present invention include, for example, polysorbate surfactants such as polysorbate-20, polysorbate-80, polysorbate-60, or polysorbate-40; Pluronic, etc. In a preferred embodiment, the liquid formulation of the present invention comprises polysorbate-80 as the surfactant.

The amount of surfactant contained in the antibody formulation of the present invention may vary depending on the specific intended properties of the formulation, the specific environment, and the specific purpose for which the formulation is used. In some preferred embodiments, the formulation may contain about 0.1 mg/mL to about 1 mg/mL, preferably about 0.2 mg/mL to about 0.8 mg/mL, for example about 0.2 mg/mL, about 0.3 mg/mL, about 0.4 mg/mL, about 0.5 mg/mL, about 0.6 mg/mL, about 0.7 mg/mL, about 0.8 mg/mL of a polysorbate surfactant (e.g., polysorbate-80). Preferably, the formulation comprises about 0.1 mg/mL to about 0.5 mg/mL of polysorbate-80, more preferably about 0.3 mg/mL of polysorbate-80.

### (v) Other excipients

The liquid formulation of the present invention may or may not comprise other excipients. For example, the liquid formulation of the present invention may further comprise a chelating agent, such as EDTA, preferably at a concentration of about 0.01 mg/mL to about 0.1 mg/mL, more preferably about 0.01 mg/mL to about 0.05 mg/mL, for example about 0.01 mg/mL, about 0.02 mg/mL, about 0.03 mg/mL, about 0.04 mg/mL, or about 0.05 mg/mL. Alternatively, the liquid formulation of the present invention may further comprise a tonicifier. The tonicifier may be selected from the group consisting of: sodium acetate, sodium lactate, sodium chloride, potassium chloride, and calcium chloride.

These and other known pharmaceutical excipients and/or additives suitable for the formulation of the present invention are well-known in the art, for example, as listed in "The Handbook of Pharmaceutical Excipients, 4th Edition, edited by Rowe et al., American Pharmaceuticals Association (2003); and Remington: the Science and Practice of Pharmacy, 21st Edition, edited by Gennaro, Lippincott Williams & Wilkins (2005)".

### II. Use of the formulation

The formulation of the present invention comprising the immunoconjugate of the present invention can be used for treating or preventing cancer in a subject. In some embodiments, the cancer may be a solid tumor or a hematologic tumor. In some embodiments, the cancer is a gastrointestinal tumor or melanoma, such as colon cancer or colorectal cancer. In some embodiments, the tumor or cancer is resistant to known drugs, such as known anti-PD-1 antibodies, for example, refractory tumors or cancers. In some embodiments, the cancer is characterized by elevated protein levels and/or nucleic acid levels (e.g., increased expression) of PD-1, PD-L1, and/or PD-L2. The present invention also provides the use of the formulation of the invention in the manufacture of a medicament for delivering the immunoconjugate according to the invention to a mammal or for treating, preventing, or ameliorating one or more of the aforementioned cancers. Preferably, the mammal is a human.

The formulation of the invention can be administered to a subject or patient via various routes. For example, administration can be performed via infusion or by syringe. Thus, in one aspect, the invention provides a pharmaceutical container (e.g., an injection container) and a delivery device (e.g., a syringe, such as a prefilled syringe) comprising the formulation of the invention. Through such administration, the patient will receive an effective amount of the conjugate of the invention, i.e., an amount sufficient to treat, ameliorate, or prevent the targeted disease or disorder.

The therapeutic effect may include reduction of physiological symptoms. The optimal effective amount and concentration of the immunoconjugate for any particular subject will depend on various factors, including the patient's age, body weight, health status and/or gender, the nature and extent of the disease, the activity of the specific immunoconjugate, the body's clearance rate thereof, and also including any other possible treatments administered in combination with said formulation. For specific circumstances, the effective amount delivered can be determined within the clinician's judgment.

The following examples are described to aid in the understanding of the present invention. The examples are not intended and should not be construed in any way to limit the scope of protection of the present invention.

**Table 1. Abbreviations and Full Names**

| Abbreviation | Full Name |
|---|---|
| BP | British Pharmacopoeia |
| ChP | Chinese Pharmacopoeia |
| Ph. Eur., EP | European Pharmacopoeia |
| NF | United States National Formulary |
| DP | Final Product/Formulation |
| DS | Drug Substance |
| ELISA | Enzyme-Linked Immunosorbent Assay |
| F | Formulation |
| HPLC-FLD | Fluorescence Detector High Performance Liquid Chromatograph |
| min | Minute |
| h | Hour |
| w | Week |
| m | Month |
| iCIEF | Imaging Capillary Isoelectric Focusing |
| IND | Investigational New Drug Application |
| JP | Japanese Pharmacopoeia |
| nrCE-SDS | Non-Reduced Sodium Dodecyl Sulfate Capillary Gel Electrophoresis |
| SEC-HPLC | Size-Exclusion High-Performance Liquid Chromatography |
| USP | United States Pharmacopeia |

### Examples

To develop a long-term stable storage formulation of αPD-1/IL2m immunoconjugate injection that ensures quality control throughout the shelf life (at least 24 months), formulation screening experiments were designed to investigate the effects of different buffer pH values, buffer systems, and excipients on the stability of the αPD-1/IL2m immunoconjugate formulation. The materials and methods used in the experiments are as follows:

### Materials and Methods

1.1. The immunoconjugate samples used in the formulation study of the present invention According to PCT Application No. PCT/CN2022/120265, the αPD-1/IL2mut immunoconjugate protein 2149 was prepared and purified.

Specifically, the heavy chain (SEQ ID NO:1) and light chain (SEQ ID NO: 2) of the anti-PD-1 antibody, as well as the IL-2m-Fc polypeptide chain (SEQ ID NO: 3, formed by connecting the IL-2m polypeptide to the N-terminus of the IgG1 Fc Knob chain via a linker), were each constructed into the pcDNA3.1 vector. A mixture of the constructed three-plasmid vectors and PEI at a 3:1 mass ratio was used for co-transfection of HEK293 cells (e.g., to transfect 3 mL of HEK293 cells, 1 µg of heavy chain + 1 µg of light chain + 1 µg of IL-2m-Fc polypeptide chain + 9 µg of PEI are required). The supernatant containing the expressed immunoconjugate protein was harvested from the culture of transfected cells and purified. Table 2 below lists the information of the immunoconjugate samples used in the following examples.

**Table 2. Sample Information**

| Sample name | Scale | Source | Concentration | Application |
|---|---|---|---|---|
| 2149-CEX 40HS 3A3-3B3 | Pilot test | Purification | 12.02 mg/mL | Formulation study |
| 2149 UFDF 20210804 | Pilot test | Purification | 9.49 mg/mL | Formulation confirmation experiment |
| 2149 UFDF 20210804 | Pilot test | Purification | 9.49 mg/mL | Study on formulation process parameters |

1.2. Reagents and materials used in the formulation study of the present invention

**Table 3. Reacent Information**

| Name | Grade | Origin and brand | Catalog No. | Specifications |
|---|---|---|---|---|
| Histidine | Pharmaceutical grade | Merck KGaA | 1.04352.1000 | Ph. Eur., JP, USP |
| L-Histidine hydrochloride | Pharmaceutical grade | Merck KGaA | 1.04354.0500 | Ph. Eur., BP, JP |
| Sucrose | Pharmaceutical grade | Merck KGaA | 1.00892.9050 | Ph. Eur., BP, ChP, JP, NF |
| L-Arginine | Pharmaceutical grade | Merck KGaA | 1.01587.1000 | USP, Ph. Eur., JP |
| Disodium edetate (EDTA) | Pharmaceutical grade | Merck KGaA | 1.37004.1000 | Ph. Eur., BP, ChP, JP, USP, ACS |
| Methionine | Pharmaceutical grade | Hubei Provincial Bafeng Pharmaceuticals & Chemicals Share Co., Ltd. | N/A | ChP |
| Polysorbate-80 | Pharmaceutical grade | Nanjing Well Pharmaceutical Technology Co., Ltd. | N/A | ChP |

| | | | | |
|---|---|---|---|---|
| Note: N/A means not applicable.Table 4 Material Information | | | | |

**Table 4. Material Information**

| Name | Origin and brand | Catalog No. |
|---|---|---|
| 15 mL Ultrafiltration centrifuge tube | Merck, USA | UFC903024 |
| Minisart high-throughput syringe filter | Sartorius, Germany | 16541-K |
| 2R neutral borosilicate glass vial | Schott Glass Technology (Suzhou) Co., Ltd. | 1142144 |
| 13 mm ETFE laminated bromobutyl rubber stopper for injectable solution | West Pharmaceutical Services, Inc. (Germany) | 7002-4373 |
| 13 mm Aluminum-plastic combination cap for antibiotic vials | West Pharmaceutical Services, Inc. (India) | 5413-2147 |

### 1.3. Instruments and equipment used in the formulation study of the present invention

**Table 5. Instrument and Equipment Information**

| Name | Origin and brand | Model | Equipment number |
|---|---|---|---|
| Constant temperature and humidity chamber | Binder (Germany) | KBFP720 | PD-A1-069 |
| | | | QC-A1-086 |
| Biochemical incubator | Shanghai Jinghong | SHP-150 | PD-A1-200 |
| Medical refrigerator | Qingdao Haier | HYC-360 | PD-A1-009 |
| Haier refrigerator (-20°C) | Qingdao Haier | BCD-290W | PD-A1-014 |
| Low-temperature refrigerator (-40°C) | Qingdao Aucma | DW-40L525 | SZ-C14-SC-REF04 |
| | | | SZ-C14-SC-REF05 |
| Oscillator | VWR (USA) | DVX-2500 | PD-A1-140 |
| Clarity tester | Tianjin Tianda Tianfa | YB-2 | PD-A1-033 |
| UV/Visible spectrophotometer | Unchained Labs (USA) | Biglunatic | AS-A1-143 |
| Luxmeter | Taiwan TES | 1330A | ENG-M1-295 |
| SevenCompact pH meter | Mettler Toledo (Switzerland) | S220 | AS-A1-029 |
| Benchtop refrigerated centrifuge | Thermo (USA) | SL16R | PD-A1-082 |
| Peristaltic pump | Watson Marlow (UK) | 520S | PD-A1-225 |
| Filling machine | Flexicon (Denmark) | FP50 | PD-C14-115 |
| Liquid particle counting system | Beckman (USA) | HIAC9703⁺ | AS-A1-166 |

### 1.4. Test items and methods for formulation stability

The following items were tested for the immunoconjugate formulation: (1) Appearance and visible foreign matter; (2) Protein content in the formulation determined by UV method; (3) Sub-visible particle content in the formulation; (4) pH value of the formulation; (5) Purity of the formulation determined by size-exclusion high-performance liquid chromatography (SEC-HPLC), expressed as the percentage of the immunoconjugate monomer peak area relative to the total peak areas; (6) Purity of the formulation determined by non-reduced capillary electrophoresis sodium dodecyl sulfate (nrCE-SDS), expressed as the percentage of the immunoconjugate monomer peak area relative to the total peak areas; (7) Charge heterogeneity in the formulation determined by imaging capillary isoelectric focusing (iCIEF), expressed as percentages of main species, acidic species, and basic species; (8) Polysorbate-80 content in the formulation determined by high-performance liquid chromatography with fluorescence detection (HPLC-FLD); (9) Relative binding activity of the immunoconjugate to PD-1 antigen and IL-2R in the formulation determined by immunoassay, such as direct ELISA.

### Test for visible particles

According to the method recorded in the Pharmacopoeia of the People's Republic of China (2015 Edition, Volume IV, General Rule 0904 "Test for Visible Particles", Beijing, China Medical Science Press. 2015) by the Chinese Pharmacopoeia Commission, visible particles in samples were inspected using a clarity tester (produced by Tianjin Tianda Tianfa, model: YB-2).

### Sub-visible particle determination

The content of sub-visible particles in the sample was determined by the light obscuration method.

### Protein content determination

The protein content in the sample was determined using a UV/Visible spectrophotometer (Unchained Labs, USA, model: Biglunatic).

### pH value determination

The pH value of the sample was determined using a SevenCompact pH meter (Mettler Toledo, Switzerland, model: S220).

### Purity determination (SEC-HPLC method)

Size exclusion chromatography was performed using a TSKgel G3000Swx1 analytical column (7.8×300 mm, 5 µm) with a TSKgel guardcolumn Swxl (6.0×40 mm, 7 µm). The mobile phase consisted of 20 mmol/L phosphate buffer + 400 mmol/L NaClO₄, pH 6.8. The injection tray temperature was 10°C, column temperature was 25°C, injection volume was 50 µL, flow rate was 0.5 mL/min, acquisition time was 30 minutes, using an FLD fluorescence detector with PMT value of 7, excitation wavelength of 285 nm, and emission wavelength of 340 nm.

The test sample was diluted with the mobile phase to 1.0 mg/mL as the test solution. The formulation buffer was diluted using the same treatment method as above to serve as the blank solution. Fifty microliters each of the blank solution and test solution were injected into the liquid chromatograph for analysis.

### Purity determination (non-reduced CE-SDS method)

Capillary gel electrophoresis was employed for analysis. An uncoated capillary with an internal diameter of 50 µm, total length of 30.2 cm, and effective length of 20.2 cm was used. Prior to electrophoresis, the capillary column was flushed with 0.1 mol/L sodium hydroxide, 0.1 mol/L hydrochloric acid, ultrapure water, and electrophoresis gel at 70 psi. The test sample was diluted with an appropriate amount of ultrapure water to 1.0 mg/mL. 50 µL of the diluted sample was transferred to a 1.5 mL centrifuge tube, to which 45 µL of pH 6.5 sample buffer (prepared by dissolving 0.32 g of citric acid monohydrate and 2.45 g of disodium hydrogen phosphate dodecahydrate in 45 mL ultrapure water, adjusting the volume to 50 mL to obtain citrate-phosphate buffer, precisely measuring 200 µL of this buffer, adding 80 µL of 10% (w/v) sodium dodecyl sulfate solution, and diluting to 1 mL with water, followed by thorough mixing) and 5 µL of 250 mmol/L NEM solution (prepared by dissolving 62 mg of N-ethylmaleimide in 2 mL ultrapure water) were added. After thorough mixing, the solution was heated at 70±2°C for 10±2 minutes, cooled to room temperature, and transferred to a sample vial as the test solution. A blank solution was prepared by subjecting the same volume of formulation buffer as the test sample to the same procedure described above. Sample injection conditions: -5 kV for 20 seconds; separation voltage: -10 kV for 35 minutes. The capillary column temperature was maintained at 25°C, with a detection wavelength of 220 nm.

### Charge heterogeneity determination (iCIEF method)

Imaging capillary isoelectric focusing (iCIEF method) was employed for detection. The capillary had an inner diameter of 100 µm and a total length of 5 cm. Prior to electrophoresis, the capillary column was rinsed with 0.5% methylcellulose solution (hereinafter also abbreviated as MC solution) and ultrapure water, respectively. Vacuum injection was employed, with prefocusing voltage and time set at 1.5 kV for 1 Minute, focusing voltage and time at 3 kV for 8 Minutes, injection time at 55 seconds, sample tray temperature at 10°C, and detection wavelength at 280 nm. The cathodic stabilizer was a 500 mmol/L arginine solution, and 0.5% MC solution was used to reduce protein adhesion to the capillary. The test sample was concentrated, buffer-exchanged, and diluted with water to 1.0 mg/mL. Then, 20 µL of the diluted test sample solution was mixed with 77 µL of premix (premix composition: 3M urea-0.5% MC, ampholyte, cathodic stabilizer, pI Marker) to prepare the sample solution for analysis. Injection analysis was performed, and the contents of main species, acidic species, and basic species were calculated based on the area normalization method.

### High-performance liquid chromatography with fluorescence detection (HPLC-FLD)

The polysorbate 80 content in the sample was determined by HPLC-FLD method. The chromatographic column was a Knitted Reaction Coil (5 m×0.50 mm ID) from SUPELCO. The mobile phase consisted of a mixed solution containing 0.15 mol/L sodium chloride, 0.05 mol/L Tris, 5% acetonitrile, 5.0 µM NPN, and 15 ppm Brij23, with a pH of 8.0. Detection conditions: Column temperature 30°C, injection volume 10 µL, acquisition time 3 minutes, flow rate 1.5 mL/min, excitation wavelength 350 nm, emission wavelength 420 nm. After completion of the run, the polysorbate 80 content in the final product was calculated using the standard curve method.

### Bioactivity assay (direct ELISA method)

The following antigens were used:
IL-2 receptor: Recombinant Human CD25-His Avi purchased from KACTUS, Catalog No.: CD5-HM425,
PD-1: Recombinant human PD-1 purchased from Sinobiological, Catalog No.: 10377-H08H,

The relative binding activities of the IL-2 end and the anti-PD-1 end of the immunoconjugate protein were detected according to the ELISA procedures described below.

The antigens were diluted to 0.5 µg/mL with PBS, coated onto 96-well microplates at 100 µL/well, and incubated overnight at 4°C. After washing, blocking solution (2% BSA-PBST, 300 µL/well) was added and incubated at 37°C for 2 h. The immunoconjugate protein was diluted to 300 µg/mL (IL-2 receptor) or 10 µg/mL (PD-1) with 2% BSA-PBST, followed by 3-fold serial dilution to 11 concentrations. The serially diluted test samples were added to the microplate (100 µL/well) after removing the blocking solution, with negative controls containing only 100 µL of dilution buffer (2% BSA-PBST), and incubated at 37°C for 60 min. After washing, HRP-conjugated goat anti-human IgG-Fc fragment (BETHYL, USA, Catalog No.: A80-104P) diluted in 2% BSA-PBST was added as the secondary antibody (1:120,000 dilution (IL-2 receptor) or 1:100,000 dilution (PD-1), 100 µL/well), and incubated at 37°C for 30 min. After washing, 100 µL of TMB substrate solution was added to each well. After 10 min of color development, the reaction was terminated by adding 100 µL of 1 mol/L H₂SO₄ per well. The OD value at 450 nm was measured with 620 nm as the reference wavelength. Using the concentration values of the serially diluted samples as the abscissa and the OD450 nm-OD620 nm values of each gradient sample as the ordinate, the EC₅₀ was calculated by four-parameter fitting using Prism to reflect the binding activity of the immunoconjugate protein to the antigen.

### Example 1. Formulation Study

### 1.1 Experimental Protocol

Prepare a buffer containing 1.55 mg/mL histidine and 80.00 mg/mL sucrose, and adjust the pH to 5.0, 5.5, 6.0, and 6.5 with dilute hydrochloric acid, designated as F1-F4, respectively. Prepare a solution containing 1.55 mg/mL histidine, 50.00 mg/mL sucrose, and 7.49 mg/mL methionine, and adjust the pH to 5.0 with dilute hydrochloric acid, designated as F5. Prepare a solution containing 1.55 mg/mL histidine, 30.00 mg/mL sucrose, and 17.42 mg/mL arginine, and adjust the pH to 5.0 with dilute hydrochloric acid, designated as F6. Prepare a solution containing 1.55 mg/mL histidine, 50.00 mg/mL sucrose, and 7.49 mg/mL methionine, and adjust the pH to 5.0 with dilute hydrochloric acid, designated as F7. Ultrafilter and exchange 2149 protein into each buffer, adjust protein content to approximately 1.0 mg/mL, and add polysorbate-80 to a final concentration of 0.3 mg/mL. Add Disodium edetate to F7 to a final concentration of 0.03 mg/mL. Detailed formulation information is shown in Table 6. Filter and aliquot into vials, then stopper and crimp. The above samples were subjected to stability testing at 40°C±2°C, with specific protocols detailed in Table 7.

**Table 6. Alternative Formulation Information Table**

| No. | Formulation information |
|---|---|
| Formulation 1 (F1) | 10 mM of histidine, 80.00 mg/mL of sucrose, 0.3 mg/mL of polysorbate-80, pH 5.0 |
| Formulation 2 (F2) | 10 mM of histidine, 80.00 mg/mL of sucrose, 0.3 mg/mL of polysorbate-80, pH 5.5 |
| Formulation 3 (F3) | 10 mM of histidine, 80.00 mg/mL of sucrose, 0.3 mg/mL of polysorbate-80, pH 6.0 |
| Formulation 4 (F4) | 10 mM of histidine, 80.00 mg/mL of sucrose, 0.3 mg/mL of polysorbate-80, pH 6.5 |
| Formulation 5 (F5) | 10 mM of histidine, 50.00 mg/mL of sucrose, 7.49 mg/mL of methionine, 0.3 mg/mL of polysorbate-80, pH 5.0 |
| Formulation 6 (F6) | 10 mM of histidine, 30.00 mg/mL of sucrose, 17.42 mg/mL of arginine, 0.3 mg/mL of polysorbate-80, pH 5.0 |
| Formulation 7 (F7) | 10 mM of histidine, 50.00 mg/mL of sucrose, 7.49 mg/mL of methionine,0.03 mg/mL of disodium edetate, 0.3 mg/mL of polysorbate-80, pH 5.0 |

| | |
|---|---|
| Note: Adjust pH with hydrochloric acid. | |

**Table 7. Experimental Protocol**

| Experimental conditions | Sampling point | | | | Test item |
|---|---|---|---|---|---|
| | Week 0 | Week 1 | Week 2 | Week 4 | |
| 40°C±2°C | √ | √ | √ | √ | Appearance, visible particulates, protein content, pH, purity, charge heterogeneity, and polysorbate-80 content |

| | | | | | |
|---|---|---|---|---|---|
| Note: (1) √ indicates a site for sampling. (2) After sampling at the above time points, all samples were first stored at -40°C and thawed as needed for testing. | | | | | |

### 1.2 Acceptance criteria

Based on product knowledge and the precision of instruments and methods, acceptance criteria were established to determine no quality change when comparing test results with initial values. For details, refer to Table 8.

**Table 8. Acceptance Criteria for No Quality Change**

| Test item | Acceptance criteria for no quality change |
|---|---|
| Appearance (observation method) | Clear to light opalescent, colorless to light yellow liquid, no foreign matter |
| Visible particles (visible particle detection method) | No foreign matter |
| Protein content (UV method) | Change rate ≤10% |
| pH value (pH value determination method) | Change value ≤0.3 |
| Purity (SEC-HPLC method) | Monomer purity change value ≤1.0% |
| Purity (non-reduced CE-SDS method) | Monomer purity change value ≤2.0% |
| Charge heterogeneity (iCIEF method) | The change in main species and acidic/basic species ≤3.0% |
| Polysorbate-80 content (HPLC-FLD) | 0.1 mg/mL-0.5 mg/mL |
| Biological activity* | 70-130% |

| | |
|---|---|
| Note: "*" indicates this standard is only applicable for formulation confirmation experiments. | |

### 1.3 Experimental results

Formulation study results are detailed in Table 9, with trend changes shown in Figure 1. Results showed that after 4 weeks at 40°C±2°C, all formulation samples met appearance and visible particle requirements; no significant changes were observed in protein content or pH. As formulation pH increased, SEC aggregate content rose while main peak content decreased more significantly. As the formulation pH decreased, the content of non-reduced CE fragments increased, and the main peak content decreased slightly more. For all samples, the charge heterogeneity of acidic species increased significantly, the main component decreased significantly, and the basic components increased noticeably. Among F1-F4, F1 performed better. Comparing F1 and F5-F7, the polysorbate-80 content in F1 and F6 decreased significantly. The purity and charge heterogeneity changes in F6 were more pronounced. Based on the combined results of purity and charge heterogeneity stability, F5 was selected for formulation confirmation experiments.

**Table 9. Formulation Study Results**

| Test item | | Time | Sample name | | | | | | |
|---|---|---|---|---|---|---|---|---|---|
| | | | F1 | F2 | F3 | F4 | F5 | F6 | F7 |
| Appearance (observation method) | | Week 0 | Pass | Pass | Pass | Pass | Pass | Pass | Pass |
| | | Week 1 | Pass | Pass | Pass | Pass | Pass | Pass | Pass |
| | | Week 2 | Pass | Pass | Pass | Pass | Pass | Pass | Pass |
| | | Week 4 | Pass | Pass | Pass | Pass | Pass | Pass | Pass |
| Visible foreign matters (visible foreign matter inspection method) | | Week 0 | Pass | Pass | Pass | Pass | Pass | Pass | Pass |
| | | Week 1 | Pass | Pass | Pass | Pass | Pass | Pass | Pass |
| | | Week 2 | Pass | Pass | Pass | Pass | Pass | Pass | Pass |
| | | Week 4 | Pass | Pass | Pass | Pass | Pass | Pass | Pass |
| Protein content (UV method, mg/mL) | | Week 0 | 1.0 | 1.0 | 1.0 | 1.0 | 1.0 | 1.0 | 1.0 |
| | | Week 1 | 1.0 | 1.0 | 1.0 | 1.0 | 1.0 | 1.0 | 1.0 |
| | | Week 2 | 1.0 | 1.0 | 1.0 | 1.0 | 1.0 | 1.0 | 1.0 |
| | | Week 4 | 1.0 | 1.0 | 1.0 | 1.0 | 1.0 | 1.0 | 1.0 |
| pH value (pH determination method) | | Week 0 | 5.1 | 5.7 | 6.1 | 6.6 | 5.0 | 5.0 | 5.0 |
| | | Week 1 | 5.1 | 5.7 | 6.1 | 6.7 | 5.0 | 5.0 | 5.0 |
| | | Week 2 | 5.1 | 5.7 | 6.1 | 6.7 | 5.1 | 5.0 | 5.1 |
| | | Week 4 | 5.2 | 5.7 | 6.1 | 6.7 | 5.1 | 5.0 | 5.1 |
| Purity | Monomer | Week 0 | 98.8 | 98.4 | 98.1 | 97.5 | 98.6 | 98.9 | 98.5 |
| (SEC-HPLC, %) | | Week 1 | 98.1 | 97.9 | 97.8 | 96.4 | 99.3 | 99.1 | 99.2 |
| | | Week 2 | 97.5 | 97.6 | 97.2 | 96.0 | 97.3 | 97.3 | 97.2 |
| | | Week 4 | 96.5 | 96.8 | 96.3 | 94.8 | 95.1 | 66.0 | 95.2 |
| | Polymer | Week 0 | 1.2 | 1.5 | 1.9 | 2.5 | 1.4 | 0.9 | 1.5 |
| | | Week 1 | 0.6 | 0.8 | 1.1 | 2.1 | 0.5 | 0.6 | 0.6 |
| | | Week 2 | 0.5 | 0.7 | 1.1 | 2.3 | 1.1 | 0.7 | 1.2 |
| | | Week 4 | 0.7 | 0.7 | 1.4 | 2.8 | 2.5 | 33.1 | 2.5 |
| Purity (nrCE-SDS, %) | Monomer | Week 0 | 97.8 | 97.9 | 97.9 | 97.9 | 97.9 | 97.8 | 97.8 |
| | | Week 1 | 96.9 | 97.3 | 97.0 | 97.1 | 97.0 | 95.6 | 97.1 |
| | | Week 2 | 95.9 | 96.1 | 96.2 | 96.4 | 96.3 | 92.7 | 96.3 |
| | | Week 4 | 95.6 | 96.5 | 96.7 | 97.2 | 97.5 | 84.2 | 97.6 |
| | Fragment | Week 0 | 2.1 | 2.0 | 2.0 | 2.0 | 2.0 | 2.1 | 2.0 |
| | | Week 1 | 3.0 | 2.6 | 2.9 | 2.8 | 2.9 | 4.3 | 2.8 |
| | | Week 2 | 4.1 | 3.9 | 3.8 | 3.5 | 3.6 | 7.2 | 3.6 |
| | | Week 4 | 4.4 | 3.5 | 3.3 | 2.8 | 2.5 | 14.5 | 2.4 |
| Charge heterogeneity (iCIEF, %) | Acidic species | Week 0 | 18.3 | 19.5 | 19.1 | 19.0 | 18.8 | 19.4 | 18.4 |
| | | Week 1 | 23.6 | 24.1 | 23.9 | 26.9 | 23.8 | 22.6 | 24.9 |
| | | Week 2 | 30.9 | 29.9 | 31.0 | 34.8 | 30.8 | 24.2 | 31.1 |
| | | Week 4 | 37.8 | 35.8 | 39.1 | 38.9 | 36.7 | 46.7 | 37.0 |
| | Main species | Week 0 | 77.9 | 77.4 | 77.2 | 77.5 | 77.9 | 76.9 | 78.2 |
| | | Week 1 | 72.0 | 72.0 | 72.9 | 70.6 | 71.6 | 72.4 | 70.9 |
| | | Week 2 | 61.6 | 63.8 | 63.3 | 61.8 | 62.6 | 64.9 | 62.1 |
| | | Week 4 | 53.3 | 56.8 | 54.7 | 55.2 | 52.7 | 36.0 | 52.6 |
| | Basic species | Week 0 | 3.8 | 3.2 | 3.7 | 3.6 | 3.3 | 3.8 | 3.3 |
| | | Week 1 | 4.3 | 3.9 | 3.2 | 2.6 | 4.5 | 5.0 | 4.3 |
| | | Week 2 | 7.5 | 6.3 | 5.7 | 3.4 | 6.6 | 10.9 | 6.8 |
| | | Week 4 | 9.0 | 7.3 | 6.2 | 5.9 | 10.6 | 17.3 | 10.4 |
| Polysorbate-80 content (FLD-HPLC, mg/mL) | | Week 0 | 0.3 | 0.3 | 0.3 | 0.3 | 0.3 | 0.3 | 0.3 |
| | | Week 1 | 0.3 | 0.3 | 0.3 | 0.3 | 0.3 | 0.3 | 0.3 |
| | | Week 2 | 0.2 | 0.3 | 0.3 | 0.3 | 0.3 | 0.1 | 0.3 |
| | | Week 4 | 0.1 | 0.2 | 0.3 | 0.3 | 0.3 | 0.0 | 0.3 |

### Example 2. Formulation Confirmation Study

### 2.1 Experimental Protocol

Based on the F5 formulation selected from the formulation screening study, this experiment primarily investigates the effects of excipients (sucrose, histidine, polysorbate-80, and methionine) on the stability of Protein 2149. Detailed formulation information is shown in Table 10, where sucrose content is appropriately increased to adjust the formulation osmolality.

Prepare the formulation buffer according to Table 10, ultrafilter and exchange Protein 2149 into the formulation solution, and adjust the protein content to 1.0 mg/mL; add polysorbate-80 to achieve a final concentration of 0.3 mg/mL; filter and fill into vials, then stopper and crimp. The above samples are subjected to stability studies under the conditions listed in Table 11.

**Table 10. Alternative Information Form**

| Formulation information |
|---|
| 10 mM of histidine, 70 mg/mL of sucrose, 7.49 mg/mL of methionine, 0.3 mg/mL of polysorbate-80, pH 5.0 |

| |
|---|
| Note: Adjust pH with hydrochloric acid. |

**Table 11. Stability Study Protocol**

| Experiment name | Experimental protocol and sampling point |
|---|---|
| High temperature stability | The samples were kept at 40°C ± 2°C and sampled on Day 0, Week 1, Week 2 and Week 4. |
| Accelerated stability | Samples stored at 25°C±2°C, sampled at 4 weeks, 2 months, and 3 months. |
| Long-term stability | Samples stored at 5°C±3°C, sampled at 3 months. |
| Agitation test | 650 r/min, room temperature, protected from light, sampling on Day 0 and 3. |
| Freeze-thaw test | One cycle consists of freezing at -20°C for 1 day and thawing at 5°C±3°C, with sampling at the 0th, 3rd, and 6th cycles. |

| | |
|---|---|
| Note: All samples taken at the above time points were first placed in a refrigerator at -40°C to be cryopreserved for testing, and then thawed and sent for testing as required. | |

### 2.2 Acceptance criteria

Specific acceptance criteria are shown in Table 8.

### 2.3 Experimental results

The results of the formulation confirmation stability study are detailed in Tables 12 and 13, with the trend changes shown in Figure 2. The results showed that the appearance and visible particles of all samples at various time points met the criteria, with no significant changes in protein content and pH. The SEC main peak decreased by 1.0% under high-temperature conditions, while the charge heterogeneity of acidic species increased under accelerated and high-temperature conditions. The charge heterogeneity of main species decreased under accelerated and high-temperature conditions, and the charge heterogeneity of basic species slightly increased under high-temperature conditions. The polysorbate-80 content remained unchanged. The IL-2 activity showed a significant decrease after 4 Weeks at high temperature, while the PD-1 activity showed no significant change. No significant changes in protein quality were observed under agitation or freeze-thaw conditions.

| Test item | | Time | | | | | | | |
|---|---|---|---|---|---|---|---|---|---|
| | | Week 0 | 40°C 1 Week | 40°C 2 Week | 40°C 4 Week | 25°C 4 Week | 25°C 2 Months | 25°C 3 Months | 2-8°C 3 Month s |
| Appearance | | Pass | Pass | Pass | Pass | Pass | Pass | Pass | Pass |
| Visible particles | | Pass | Pass | Pass | Pass | Pass | Pass | Pass | Pass |
| Protein content (UV, mg/mL) | | 1.0 | 1.0 | 1.0 | 1.0 | 1.0 | 1.0 | 1.1 | 1.1 |
| pH value | | 5.1 | 5.1 | 5.1 | 5.1 | 5.1 | 5.0 | 5.0 | 5.0 |
| Purity (SEC-HPLC, %) | Monomer | 97.1 | 97.1 | 96.8 | 96.1 | 96.8 | 97.8 | 97.4 | 97.8 |
| | Polymer | 1.3 | 1.3 | 1.4 | 1.6 | 1.2 | 0.8 | 0.9 | 0.9 |
| Purity (nrCE-SDS, %) | Monomer | 98.9 | 98.5 | 97.0 | 96.1 | 97.7 | 96.9 | 96.7 | 98.3 |
| | Fragment | 1.0 | 1.4 | 2.9 | 3.7 | 2.0 | 2.6 | 2.6 | 1.3 |
| Charge heterogeneity (iCIEF,%) | Acidic species | 24.2 | 28.9 | 31.2 | 37.8 | 22.1 | 26.4 | 31.5 | 24.7 |
| | Main species | 73.0 | 67.2 | 63.9 | 55.3 | 73.9 | 69.8 | 63.6 | 71.7 |
| | Basic species | 2.8 | 3.9 | 5.0 | 6.9 | 4.0 | 3.8 | 5.0 | 3.6 |
| Polysorbate-80 content (HPLC-FLD, mg/mL) | | 0.3 | 0.3 | 0.3 | 0.3 | 0.3 | 0.3 | 0.3 | 0.3 |
| Biological activity (ELISA, %) | IL2 | 106 | 107 | 111 | 64 | 114 | 102 | 82 | 82 |
| | PD-1 | 104 | 100 | 106 | 93 | 91 | 79 | 100 | 106 |

As shown in Table 12, after 4 weeks at 40°C±2°C, the SEC main peak decreased by 1.0%, the non-reduced CE-SDS main peak decreased by 2.8%, the charge heterogeneity of acidic species increased by 13.6%, the main species decreased by 17.7%, and the basic species slightly increased. After 3 months at 25°C±2°C, the non-reduced CE-SDS decreased by 2.2%, the charge heterogeneity of acidic species increased by 7.3%, and the main species decreased by 9.4%. No significant changes were observed in any test items after long-term storage for 3 months at 5°C±3°C.

**Table 13. Results of Agitation and Freeze-Thaw Studies**

| Test item | | Time | | |
|---|---|---|---|---|
| | | Week 0 | 6 Freeze-thaw cycles | 3 Days of agitation |
| Appearance | | Pass | Pass | Pass |
| Visible particles | | Pass | Pass | Pass |
| Sub-visible particles (light obscuration method, particles/mL) | ≥2 µm | 44 | 112 | 64 |
| | ≥5 µm | 10 | 14 | 12 |
| | ≥10 µm | 4 | 3 | 4 |
| | ≥25 µm | 0 | 0 | 2 |
| Protein content (UV method, mg/mL) | | 1.0 | 1.0 | 1.0 |
| pH value | | 5.1 | 5.1 | 5.1 |
| Purity (SEC-HPLC, %) | Monomer | 97.1 | 96.3 | 97.3 |
| | Polymer | 1.3 | 2.1 | 1.1 |
| Purity (nrCE-SDS, %) | Monomer | 98.9 | 98.9 | 99.0 |
| | Fragment | 1.0 | 1.0 | 0.9 |
| Charge heterogeneity (iCIEF, %) | Acidic species | 24.2 | 22.3 | 23.4 |
| | Main species | 73.0 | 75.3 | 74.0 |
| | Basic species | 2.8 | 2.4 | 2.7 |
| Polysorbate-80 content (HPLC-FLD, mg/mL) | | 0.3 | 0.3 | 0.3 |
| Biological activity (ELISA, %) | IL2 | 106 | 112 | 116 |
| | PD-1 | 104 | 106 | 105 |

### Conclusion

Based on the formulation study results and formulation confirmation experimental results, the confirmed formulation was selected as the final 2149 formulation. To avoid using hydrochloric acid for pH adjustment during production, the buffer system was adjusted to histidine and histidine hydrochloride. The final 2149 formulation is as follows: 1.0 mg/mL recombinant αPD-1/IL2m immunoconjugate protein, 0.12 mg/mL histidine, 1.94 mg/mL histidine hydrochloride, 70.00 mg/mL sucrose, 7.49 mg/mL methionine, and 0.3 mg/mL polysorbate-80, pH 5.0.

### Example 3. In vivo pharmacodynamic study of αPD-1/IL2m immunoconjugate

The αPD-1/IL2m immunoconjugate of the present invention can specifically bind to human PD-1 at its αPD-1 end to block the binding of PD-1 to PD-L1, thereby releasing the immune brake mechanism, while at its IL-2m end, it can specifically bind to the IL-2 receptor on T cells or NK cells, thereby activating and expanding T or NK cells to enhance the effect of PD-1 antibody immunotherapy.

To demonstrate the in vivo efficacy of the αPD-1/IL2m immunoconjugate of the present invention, MC38 cells (mouse colon cancer cell line, Shanghai HeYuan Biology) were inoculated into hPD-1 knock-in mice to determine the anti-tumor efficacy of the immunoconjugate (2149) of the present invention. The experiment used SPF-grade female hPD-1 knock-in mice (purchased from Shanghai Model Organisms Center, Inc.), with a certificate number of NO. 20170010006762.

MC38 cells were routinely passaged for subsequent in vivo experiments. The cells were collected by centrifugation and resuspended in PBS (1×) to prepare a cell suspension with a concentration of 5×10⁶ cells/mL. On Day 0, 0.2 mL of the cell suspension was subcutaneously inoculated into the right abdominal region of hPD-1 knock-in mice to establish the MC38 tumor-bearing mouse model.

Eight days after tumor cell inoculation, the tumor volume of each mouse was measured, and grouping was performed (8 mice per group). The dosing regimen and administration method are shown in Table 14.

**Table 14: Grounine Dosing Regimen. and Administration Method for In Vivo Experiments**

| Group | Administrated dose | Number of administration | Administration mode |
|---|---|---|---|
| h-IgG* | 40 mg/kg | QW × 3 | Intraperitoneal injection |
| 2149, 10 mg/kg | 10 mg/kg | QW × 3 | Intraperitoneal |
| | | | injection |
| 2149, 20 mg/kg | 20 mg/kg | QW × 3 | Intraperitoneal injection |
| 2149, 40 mg/kg | 40 mg/kg | QW × 3 | Intraperitoneal injection |

| | | | |
|---|---|---|---|
| *: h-IgG is the isotype control antibody, purchased from Equitech-Bio, batch number 161206-0656. | | | |

The concentrations of h-IgG, 2149 at 10 mg/kg, 2149 at 20 mg/kg, and 2149 at 40 mg/kg were 4 mg/mL, 1 mg/mL, 2 mg/mL, and 4 mg/mL, respectively, administered once weekly for a total of three times (QW × 3). Administration was performed on Day 8, 15, and 22 after MC38 cell inoculation, with tumor volume and body weight monitored twice weekly, as shown in Figures 3A-B. Mice with tumors exceeding 2000 mm³ were euthanized. A subset of mice was monitored until Day 61. Since some mice in certain groups were euthanized due to tumor volumes exceeding 2000 mm³, the relative tumor growth inhibition rate (TGI%) was calculated based on Day 36 post-inoculation using the following formula: TGI% = 100% × (Tumor volume of control group - Tumor volume of treatment group) / (Tumor volume of control group - Tumor volume of control group before treatment). Tumor volume measurement: the maximum length axis (L) and the maximum width axis (W) of each tumor were measured using a vernier caliper, and the tumor volume was calculated according to the following formula: V=L*W²/2. The body weight was measurement using an electronic balance.

Tumor growth curves and survival curves are shown in Figures 3A and 3B. The anti-tumor effects of molecule 2149 exhibited dose-dependency across different dose groups, with complete tumor regression observed in the 20 mg/kg and 40 mg/kg groups. This advantage was also reflected in the survival curve shown in Figure 3B, where 100% tumor regression was achieved in these two groups, whereas only 2 out of 8 mice showed complete tumor regression in the 10 mg/kg group. Tumor inhibition rates are presented in Table 15: On Day 36 post-inoculation, compared to the h-IgG group, the tumor inhibition rates for 2149 10 mg/kg, 2149 20 mg/kg, and 2149 40 mg/kg were 84%, 103%, and 103%, respectively. Concurrently, body weight measurements (Figure 3C) showed no significant differences among groups on Day 36 post-inoculation.

**Table 15. Statistical Analysis of Anti-tumor Efficacy**

| Group | Tumor volume (mm³) | Tumor inhibition rate (%) @ Day17 | Complete response rate* @ Day36 |
|---|---|---|---|
| h-IgG | 2426.63 | N/A | 0/8 |
| 2149, 10 mg/kg | 458.62 | 84 | 2/8 |
| 2149, 20 mg/kg | 0 | 103 | 8/8 |
| 2149, 40 mg/kg | 0 | 103 | 8/8 |

| | | | |
|---|---|---|---|
| *Complete response rate: Complete tumor regression with tumor volume of 0. | | | |

To demonstrate the in vivo efficacy of immunoconjugate 2149, B16F10 cells resistant to PD-1 antibody (mouse melanoma cell line, ATCC CRL-6475) were inoculated into hPD-1 knock-in mice to evaluate the anti-tumor efficacy of the bifunctional PD-1 antibody and IL-2 mutant molecule fusion protein (2149) of the present invention. The experiment used SPF-grade female hPD-1 knock-in mice (purchased from Shanghai Model Organisms Center, Inc.), with a certificate number of NO. 20170010007909.

B16F10 cells were routinely passaged for subsequent in vivo experiments. The cells were collected by centrifugation and resuspended in PBS (1×) to prepare a cell suspension with a concentration of 2.5×10⁶ cells/mL. On Day 0, 0.2 mL of the cell suspension was subcutaneously inoculated into the right abdominal region of hPD-1 knock-in mice to establish the B16F10 tumor-bearing mouse model.

Six days after tumor cell inoculation, the tumor volume of each mouse was measured, and grouping was performed (8 mice per group).The dosing regimen and administration method are shown in Table 16.

| Group | Administrated dose | Number of administration | Administration mode |
|---|---|---|---|
| h-IgG* | 40 mg/kg | QW × 3 | Intraperitoneal injection |
| IBI308^{&}, 20 mg/kg | 20 mg/kg | QW × 3 | Intraperitoneal injection |
| IBI308, 40 mg/kg | 40 mg/kg | QW × 3 | Intraperitoneal injection |
| 2149, 20 mg/kg | 20 mg/kg | QW × 3 | Intraperitoneal injection |
| 2149, 40 mg/kg | 40 mg/kg | QW × 3 | Intraperitoneal injection |

| | | | |
|---|---|---|---|
| *: h-IgG is the isotype control antibody, purchased from Equitech-Bio, batch number 161206-0656; ^{&}: IBI308 is the parental anti-PD-1 monoclonal antibody (Sintilimab). | | | |

The concentrations used for h-IgG, IBI308 at 20 mg/kg, IBI308 at 40 mg/kg, 2149 at 20 mg/kg, and 2149 at 40 mg/kg were 4 mg/mL, 2 mg/mL, 4 mg/mL, 2 mg/mL, and 4 mg/mL, respectively, administered once weekly for a total of three doses (QW × 3). Administration was performed on Days 8, 15, and 22 post-B16F10 cell inoculation, with tumor volume and body weight monitored twice weekly until Day 22, as shown in Figures 4A-B. Due to the high metastatic potential of B16F10 cells leading to mouse mortality, the relative tumor inhibition rate (TGI%) was calculated based on Day 15 post-inoculation using the formula: TGI% = 100% × (Tumor volume of control group - Tumor volume of treatment group) / (Tumor volume of control group - Tumor volume of control group before treatment). Tumor volume measurement: the maximum length axis (L) and the maximum width axis (W) of each tumor were measured using a vernier caliper, and the tumor volume was calculated according to the following formula: V=L*W²/2. The body weight was measurement using an electronic balance. Mice with tumors exceeding 2000 mm³ were euthanized.

Mouse tumor growth curves are shown in Figures 4A and 4B. In the PD-1 resistance model, IBI308 demonstrated minimal efficacy, whereas the 20 mg/kg and 40 mg/kg dose groups of 2149 exhibited certain anti-tumor effects, with the higher dose showing superior efficacy to the lower dose. This effect was also reflected in the survival curves, with 2 mice in the high-dose group showing complete tumor regression at the experimental endpoint (Figure 4C and Table 17).

Tumor inhibition rates are presented in Table 17: On Day 15 post-inoculation, compared to the h-IgG 40 mg/kg group, the tumor inhibition rates for IBI308 20 mg/kg, IBI308 40 mg/kg, 2149 20 mg/kg, and 2149 40 mg/kg were 29%, 27%, 82%, and 86%, respectively. Concurrently, body weight measurements (Figure 4D) showed no significant differences among groups on Day 22 post-inoculation.

**Table 17. Statistical Analvsis of Anti-tumor Efficacy**

| Group | Tumor volume (mm³) | Tumor inhibition rate (%) @Day15 | Complete response rate @Day22 |
|---|---|---|---|
| h-IgG | 1014.92 | N/A | 0/6 |
| IBI308, 20 mg/kg | 735.02 | 29 | 0/6 |
| IBI308, 40 mg/kg | 757.11 | 27 | 0/6 |
| 2149, 20 mg/kg | 227.62 | 82 | 0/6 |
| 2149, 40 mg/kg | 193.90 | 86 | 2/6 |

To demonstrate the superior in vivo efficacy of the αPD-1/IL2m immunoconjugate 2149 compared to the control drug PD-1-IL2v (molecule ID 2061, sequence source US20180326010A1, see also sequence listing), PD-1 antibody-resistant B16F10 cells (mouse melanoma cell line, ATCC CRL-6475) were inoculated into hPD-1 knock-in mice to evaluate the antitumor efficacy of the bifunctional PD-1 antibody and IL-2 mutant molecule fusion protein (2149) of the present invention. The experiment used SPF-grade female hPD-1 knock-in mice (purchased from Shanghai Model Organisms Center, Inc.), with a certificate number of NO. 20170010008942. B16F10 cells were routinely passaged for subsequent in vivo experiments. The cells were collected by centrifugation and resuspended in PBS (1×) to prepare a cell suspension with a concentration of 2.5×10⁶ cells/mL. On Day 0, 0.2 mL of the cell suspension was subcutaneously inoculated into the right abdominal region of hPD-1 knock-in mice to establish the B16F10 tumor-bearing mouse model.

Eight days after tumor cell inoculation, the tumor volume of each mouse was measured, and grouping was performed (7 mice per group). The dosing regimen and administration method are shown in Table 18.

**Table 18. Grouping, Dosing Regimen, and Administration Method for In Vivo Experiments**

| Group | Administrated dose | Number of administration | Administration mode |
|---|---|---|---|
| h-IgG* | 40 mg/kg | QW × 3 | Intraperitoneal injection |
| IBI308 | 40 mg/kg | QW × 3 | Intraperitoneal injection |
| 2061, 10mg/kg | 10 mg/kg | QW × 3 | Intraperitoneal injection |
| 2061, 20mg/kg | 20 mg/kg | QW × 3 | Intraperitoneal injection |
| 2061, 40mg/kg | 40 mg/kg | QW × 3 | Intraperitoneal injection |
| 2149, 10mg/kg | 10 mg/kg | QW × 3 | Intraperitoneal injection |
| 2149, 40mg/kg | 20 mg/kg | QW × 3 | Intraperitoneal injection |
| 2149, 40mg/kg | 40 mg/kg | QW × 3 | Intraperitoneal injection |

| | | | |
|---|---|---|---|
| *: h-IgG is the isotype control antibody, purchased from Equitech-Bio, batch number 161206-0656. | | | |

The concentrations of h-IgG, IBI308 (40 mg/kg), 2061 (10 mg/kg), 2061 (20 mg/kg), 2061 (40 mg/kg), 2149 (10 mg/kg), 2149 (20 mg/kg), and 2149 (40 mg/kg) were 4 mg/mL, 2 mg/mL, 4 mg/mL, 2 mg/mL, and 4 mg/mL, respectively, administered once weekly for a total of three doses (QW × 3). Administration was performed on Day 8, 15, and 22 post-B16F10 cell inoculation, with tumor volume and body weight monitored twice weekly until Day 33, as shown in Figure 5A. Due to the high metastatic potential of B16F10 cells leading to mouse mortality, the relative tumor inhibition rate (TGI%) was calculated on Day 19 post-inoculation using the following formula: TGI% = 100% × (Tumor volume of control group - Tumor volume of treatment group) / (Tumor volume of control group - Tumor volume of control group before treatment). Tumor volume measurement: the maximum length axis (L) and the maximum width axis (W) of each tumor were measured using a vernier caliper, and the tumor volume was calculated according to the following formula: V=L*W2/2. The body weight was measurement using an electronic balance. Mice with tumors exceeding 2000 mm³ were euthanized. If more than half of the mice in a group died, the tumor growth curve for that group was not displayed at that time point.

The tumor inhibition rate results are shown in Table 19: On Day 19 post-inoculation, compared to the h-IgG, 40 mg/kg group, the tumor inhibition rates for IBI308, 40 mg/kg, 2061, 10 mg/kg, 2149, 10 mg/kg, 2149, 20 mg/kg, and 2149, 40 mg/kg were 21%, 96%, 79%, 87%, and 97%, respectively. The TGI for the 2061, 20 mg/kg and 40 mg/kg groups was not calculated due to significant body weight loss and mouse deaths after the first dose administration.

Furthermore, statistical analysis of mouse survival (Figure 5B) revealed that, when comparing the maximum administered/tolerated doses of 2061 and 2149 in this experiment, 2149 at 40 mg/kg and 2061 at 10 mg/kg exhibited superior survival outcomes. Specifically, complete tumor regression was observed in 3 out of 7 mice treated with 2149, whereas only 1 mouse showed complete regression in the 2061 group. The results of mouse body weight monitoring (Figure 5C) showed that, on Day 29 post-inoculation, no body weight loss was observed in any 2149 dose group, whereas the 2061 group exhibited body weight loss. At the low dose (10 mg/kg), the average body weight loss exceeded 5%. At the medium (20 mg/kg) and high doses (40 mg/kg), mouse deaths occurred, with 6 out of 7 mice dying per group (details in Table 12). In contrast, 2149 was relatively safe, with no significant body weight loss at low, medium, or high doses. Only one mouse died at the low and medium doses, and no deaths occurred at the high dose. Additionally, 2149 demonstrated a higher complete tumor response rate compared to 2061 (Table 19). Therefore, 2149 exhibits superior efficacy and safety compared to 2061, with a wider therapeutic window.Table 19. Statistical Analysis of Anti-tumor Efficacy

| Group | Day 19 tumor volume (mm³) | Day 19 mouse mortality | Day 33 complete tumor response rate |
|---|---|---|---|
| h-IgG | 1934.73 | 3/7 | 0/7 |
| IBI308, 40 mg/kg | 1540.91 | 2/7 | 0/7 |
| 2061, 10 mg/kg | 146.28 | 0/7 | 1/7 |
| 2061, 20 mg/kg | N/A | 6/7 | 0/7 |
| 2061, 40 mg/kg | N/A | 6/7 | 0/7 |
| 2149, 10 mg/kg | 450.77 | 1/7 | 0/7 |
| 2149, 20 mg/kg | 301.05 | 1/7 | 1/7 |
| 2149, 40 mg/kg | 114.50 | 0/7 | 3/7 |

### Sequence listing

| **SEQ ID NO** | **Description** | **Sequence** |
|---|---|---|
| 1 | Full-length native IL-2 | MYRMQLLSCIALSLALVTNSAPTSSSTKKTQLQLEHLLLDLQMILNGINNYK NPKLTRMLTFKFYMPKKATELKHLQCLEEELKPLEEVLNLAQSKNFHLRPR DLISNINVIVLELKGSETTFMCEYADETATIVEFLNRWITFCQSIISTLT |
| 2 | Mature IL-2 | |
| 3 | Wild-type IL-2 (comprising C125S) | APTSSSTKKTQLQLEHLLLDLQMILNGINNYKNPKLTRMLTFKFYMPKKAT ELKHLQCLEEELKPLEEVLNLAQSKNFHLRPRDLISNINVIVLELKGSETTFM CEYADETATIVEFLNRWITFSQSIISTLT |
| | | |
| **Molecule 2149** | **Comprising T3A+N88R+S130R mutations and IL15 B'C' loop substitution** | |
| 4 | IL-2 mutant protein | APASSSTKKTQLQLEHLLLDLQMILNGINNYKNPKLTRMLTFKFYMPKKA TELKHLQCLEEELKPLEEVLNLAGDASIHDLISRINVIVLELKGSETTFMCE YADETATIVEFLNRWITFSQSIIRTLT |
| 5 | Linker | GGGGSGGGGS |
| 6 | Fc-knob | DKTHTCPPCPAPEAAGGPSVFLFPPKPKDTLMISRTPEVTCVVVDVSHEDP EVKFNWYVDGVEVHNAKTKPREEQYNSTYRVVSVLTVLHQDWLNGKE YKCKVSNKALPAPIEKTISKAKGQPREPQVYTLPPCRDELTKNQVSLWCL VKGFYPSDIAVEWESNGQPENNYKTTPPVLDSDGSFFLYSKLTVDKSRWQ QGNVFSCSVMHEALHNHYTQKSLSLSPGK |
| 7 | IL-2m-linker-Fc-kn ob | APASSSTKKTQLQLEHLLLDLQMILNGINNYKNPKLTRMLTFKFYMPKKA TELKHLQCLEEELKPLEEVLNLAGDASIHDLISRINVIVLELKGSETTFMCE YADETATIVEFLNRWITFSQSIIRTLTGGGGSGGGGSDKTHTCPPCPAPEA AGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPI EKTISKAKGQPREPQVYTLPPCRDELTKNQVSLWCLVKGFYPSDIAVEWE SNGQPENNYKTTPPVLDSDGSFFLYSKLTVDKSRWQQGNVFSCSVMHEA LHNHYTQKSLSLSPGK |
| 8 | Anti-PD-1 heavy chain variable region | QVQLVQSGAEVKKPGSSVKVSCKASGGTFSSYAISWVRQAPGQGLEWM GLIIPMFDTAGYAQKFQGRVAITVDESTSTAYMELSSLRSEDTAVYYCAR AEHSSTGTFDYWGQGTLVTVSS |
| 9 | Anti-PD-1 heavy chain variable region CDR1 | GGTFSSYAIS |
| 10 | Anti-PD-1 heavy chain variable region CDR2 | LIIPMFDTAGYAQKFQG |
| 11 | Anti-PD-1 heavy chain variable region CDR3 | AEHSSTGTFDY |
| 12 | Fc-hole | DKTHTCPPCPAPEAAGGPSVFLFPPKPKDTLMISRTPEVTCVVVDVSHEDP EVKFNWYVDGVEVHNAKTKPREEQYNSTYRVVSVLTVLHQDWLNGKE YKCKVSNKALPAPIEKTISKAKGQPREPQVCTLPPSRDELTKNQVSLSCAV KGFYPSDIAVEWESNGQPENNYKTTPPVLDSDGSFFLVSKLTVDKSRWQQ GNVFSCSVMHEALHNHYTQKSLSLSPGK |
| 13 | Anti-PD-1 heavy chain constant region | ASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEPK SCDKTHTCPPCPAPEAAGGPSVFLFPPKPKDTLMISRTPEVTCVVVDVSHE DPEVKFNWYVDGVEVHNAKTKPREEQYNSTYRVVSVLTVLHQDWLNG KEYKCKVSNKALPAPIEKTISKAKGQPREPQVCTLPPSRDELTKNQVSLSC |
| | | AVKGFYPSDIAVEWESNGQPENNYKTTPPVLDSDGSFFLVSKLTVDKSRW QQGNVFSCSVMHEALHNHYTQKSLSLSPGK |
| 14 | Anti-PD-1 heavy chain | QVQLVQSGAEVKKPGSSVKVSCKASGGTFSSYAISWVRQAPGQGLEWM GLIIPMFDTAGYAQKFQGRVAITVDESTSTAYMELSSLRSEDTAVYYCAR AEHSSTGTFDYWGQGTLVTVSSASTKGPSVFPLAPSSKSTSGGTAALGCL VKDYFPEPVTVSWNSGALTSGVHTFPAVLQSSGLYSLSSVVTVPSSSLGT QTYICNVNHKPSNTKVDKKVEPKSCDKTHTCPPCPAPEAAGGPSVFLFPP KPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQP REPQVCTLPPSRDELTKNQVSLSCAVKGFYPSDIAVEWESNGQPENNYKT TPPVLDSDGSFFLVSKLTVDKSRWQQGNVFSCSVMHEALHNHYTQKSLS LSPGK |
| 15 | Anti-PD-1 light chain variable region | DIQMTQSPSSVSASVGDRVTITCRASQGISSWLAWYQQKPGKAPKLLISA ASSLQSGVPSRFSGSGSGTDFTLTISSLQPEDFATYYCQQANHLPFTFGGG TKVEIK |
| 16 | Anti-PD-1 light chain variable region CDR1 | RASQGISSWLA |
| 17 | Anti-PD-1 light chain variable region CDR2 | AASSLQS |
| 18 | Anti-PD-1 light chain variable region CDR3 | QQANHLPFT |
| 19 | Anti-PD-1 light chain constant region | |
| 20 | Anti-PD-1 light chain | DIQMTQSPSSVSASVGDRVTITCRASQGISSWLAWYQQKPGKAPKLLISA ASSLQSGVPSRFSGSGSGTDFTLTISSLQPEDFATYYCQQANHLPFTFGGG TKVEIKRTVAAPSVFIFPPSDEQLKSGTASVVCLLNNFYPREAKVQWKVD NALQSGNSQESVTEQDSKDSTYSLSSTLTLSKADYEKHKVYACEVTHQG LSSPVTKSFNRGEC |
| 21 | Fc (without Knob-hole mutation, with LALA mutation) | DKTHTCPPCPAPEAAGGPSVFLFPPKPKDTLMISRTPEVTCVVVDVSHEDP EVKFNWYVDGVEVHNAKTKPREEQYNSTYRVVSVLTVLHQDWLNGKE YKCKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQQ GNVFSCSVMHEALHNHYTQKSLSLSPGK |
| 22 | Fc (without Knob-hole mutation, without LALA mutation) | DKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKE YKCKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQQ GNVFSCSVMHEALHNHYTQKSLSLSPGK |
| | | |
| **Molecule 2061** | **The control molecule in this study, derived from** US20180326010A1**, consists of an anti-PD-1 IgG antibody conjugated with an IL-2 mutant** | |
| 23 | Heavy chain knob | EVQLLESGGGLVQPGGSLRLSCAASGFSFSSYTMSWVRQAPGKGLEWVAT ISGGGRDIYYPDSVKGRFTISRDNSKNTLYLQMNSLRAEDTAVYYCVLLTG RVYFALDSWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICN VNHKPSNTKVDKKVEPKSCDKTHTCPPCPAPEAAGGPSVFLFPPKPKDTLM ISRTPEVTCVVVDVSHEDPEVKFNWYVDGVEVHNAKTKPREEQYNSTYRV VSVLTVLHQDWLNGKEYKCKVSNKALPAPIEKTISKAKGQPREPQVYTLPP CRDELTKNQVSLWCLVKGFYPSDIAVEWESNGQPENNYKTTPPVLDSDGS FFLYSKLTVDKSRWQQGNVFSCSVMHEALHNHYTQKSLSLSPGGGGGSGG GGSGGGGSAPASSSTKKTQLQLEHLLLDLQMILNGINNYKNPKLTRMLTA |
| | | KFAMPKKATELKHLQCLEEELKPLEEVLNGAQSKNFHLRPRDLISNINVIVL ELKGSETTFMCEYADETATIVEFLNRWITFAQSIISTLT |
| 24 | Heavy chain hole | EVQLLESGGGLVQPGGSLRLSCAASGFSFSSYTMSWVRQAPGKGLEWVAT ISGGGRDIYYPDSVKGRFTISRDNSKNTLYLQMNSLRAEDTAVYYCVLLTG RVYFALDSWGQGTLVTVSSASTKGPSVFPLAPSSKSTSGGTAALGCLVKDY FPEPVTVSWNSGALTSGVHTFPAVLQSSGLYSLSSVVTVPSSSLGTQTYICN VNHKPSNTKVDKKVEPKSCDKTHTCPPCPAPEAAGGPSVFLFPPKPKDTLM ISRTPEVTCVVVDVSHEDPEVKFNWYVDGVEVHNAKTKPREEQYNSTYRV VSVLTVLHQDWLNGKEYKCKVSNKALPAPIEKTISKAKGQPREPQVCTLPP SRDELTKNQVSLSCAVKGFYPSDIAVEWESNGQPENNYKTTPPVLDSDGSF FLVSKLTVDKSRWQQGNVFSCSVMHEALHNHYTQKSLSLSPGK |
| 25 | Light chain LC | DIVMTQSPDSLAVSLGERATINCKASESVDTSDNSFIHWYQQKPGQSPKLLI YRSSTLESGVPDRFSGSGSGTDFTLTISSLQAEDVAVYYCQQNYDVPWTFG QGTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC |

## Claims

1. An immunoconjugate liquid formulation comprising:
(i) an immunoconjugate,
(ii) a buffering agent,
(iii) a stabilizer, and
(iv) a surfactant,
wherein the immunoconjugate comprises: (a) an anti-PD-1 half-antibody and (b) a mutant IL2 fusion polypeptide,
wherein the anti-PD-1 half-antibody comprises an Fab fragment fused to an Fc region and comprises three heavy chain CDR sequences contained in the heavy chain variable region sequence of SEQ ID NO: 8; and three light chain CDR sequences contained in the light chain variable region sequence of SEQ ID NO: 15; and
wherein the mutant IL2 fusion polypeptide comprises a mutant IL-2 polypeptide fused to an Fc region, and the mutant IL-2 polypeptide comprises (i) N88R + S130R; and (ii) a B'C' loop sequence of AGDASIH or AQSKNFH, and optionally (iii) T3A;
wherein the liquid formulation has a pH of about 5.0 to about 6.5, for example, a pH of about 5.0, about 5.5, about 6.0, or about 6.5, preferably a pH of about 5.0.

2. The liquid formulation according to claim 1, **characterized in that** the concentration of the immunoconjugate is about 0.5 mg/mL to about 150 mg/mL, preferably about 0.5 to about 10 mg/mL, for example about 0.5 mg/mL, about 1 mg/mL, about 2 mg/mL, about 3 mg/mL, about 4 mg/mL, about 5 mg/mL, about 6 mg/mL, about 7 mg/mL, about 8 mg/mL, about 9 mg/mL, or about 10 mg/mL.

3. The liquid formulation according to claim 1 or 2, **characterized in that** the buffering agent comprises a histidine buffering agent, preferably, the buffering agent comprises about 5 mM to about 50 mM of histidine, preferably about 10 mM to about 30 mM of histidine, for example, about 10 mM, about 15 mM, about 20 mM, about 25 mM, or about 30 mM of histidine.

4. The liquid formulation according to any one of claims 1-3, **characterized in that** the stabilizer comprises:
- sugars, particularly sucrose, preferably at a concentration of about 50 mg/mL to about 120 mg/mL, for example about 80 mg/mL; or
- a combination of sugars and amino acids, particularly a combination of sucrose and methionine, preferably, the sucrose is at a concentration of about 30 mg/mL to about 100 mg/mL, for example, about 50 mg/mL, about 60 mg/mL, about 70 mg/mL, or about 80 mg/mL, and the methionine is at a concentration of about 20 mM to about 80 mM, for example about 30 mM, about 40 mM, about 50 mM, or about 60 mM.

5. The liquid formulation according to any one of claims 1-4, **characterized in that** the surfactant in the liquid formulation is selected from polysorbate surfactants, preferably polysorbate-80.

6. The liquid formulation according to any one of claims 1-5, **characterized in that** the surfactant is at a concentration of about 0.1 mg/mL to about 1 mg/mL, preferably about 0.1 mg/mL to about 0.5 mg/mL, for example about 0.1 mg/mL, about 0.2 mg/mL, about 0.3 mg/mL, about 0.4 mg/mL, or about 0.5 mg/mL.

7. The liquid formulation according to any one of claims 1-6, **characterized in that** the liquid formulation comprises EDTA, preferably at a concentration of about 0.01 mg/mL to about 0.1 mg/mL, more preferably about 0.01 mg/mL to about 0.05 mg/mL, for example about 0.01 mg/mL, about 0.02 mg/mL, about 0.03 mg/mL, about 0.04 mg/mL, or about 0.05 mg/mL.

8. The liquid formulation according to any one of claims 1-7, wherein the immunoconjugate comprises knob-in-hole mutations,
preferably wherein the Fc region of the fusion polypeptide comprises a knob mutation and the Fc region of the half-antibody comprises a hole mutation.

9. The liquid formulation according to any one of claims 1-8, wherein the anti-PD-1 half-antibody comprises a heavy chain variable region and a light chain variable region, the heavy chain variable region comprises the amino acid sequence shown in SEQ ID NO: 8 or a sequence having at least 90%, 91%, 92%, 93%, 94%, 95%, 96%, 97%, 98%, 99% or more sequence identity thereto; and the light chain variable region comprises the amino acid sequence shown in SEQ ID NO: 15 or a sequence having at least 90%, 91%, 92%, 93%, 94%, 95%, 96%, 97%, 98%, 99% or more sequence identity thereto,
preferably, the anti-PD-1 half-antibody comprises a heavy chain and a light chain, wherein the heavy chain comprises the amino acid sequence of SEQ ID NO: 14 or a sequence having at least 90%, 91%, 92%, 93%, 94%, 95%, 96%, 97%, 98%, 99% or more sequence identity thereto; and the light chain comprises the amino acid sequence of SEQ ID NO: 20 or a sequence having at least 90%, 91%, 92%, 93%, 94%, 95%, 96%, 97%, 98%, 99% or more sequence identity thereto.

10. The liquid formulation according to any one of claims 1-9, wherein the mutant IL-2 protein comprises the amino acid sequence of SEQ ID NO: 4 or a sequence having at least 90%, 91%, 92%, 93%, 94%, 95%, 96%, 97%, 98%, 99% or more sequence identity thereto;
preferably, the mutant IL-2 fusion polypeptide comprises the amino acid sequence of SEQ ID NO: 7 or a sequence having at least 90%, 91%, 92%, 93%, 94%, 95%, 96%, 97%, 98%, 99% or more sequence identity thereto.

11. The liquid formulation according to any one of claims 1-10, **characterized in that** the immunoconjugate is recombinantly expressed in HEK293 cells or CHO cells.

12. The liquid formulation according to any one of claims 1-11, **characterized in that** the liquid formulation is an injectable preparation, preferably for subcutaneous or intravenous injection, or an infusion preparation, such as for intravenous infusion.

13. The liquid formulation according to any one of claims 1-12, comprising:
(i) about 1-50 mg/mL of the immunoconjugate;
(ii) about 5-50 mM of histidine;
(iii) about 50-120 mg/mL of sucrose, or a combination of about 30-100 mg/mL of sucrose and about 20-80 mM of methionine; and
(iv) about 0.1-0.5 mg/mL of polysorbate 80;
wherein the pH of the liquid formulation is about 5.0-5.5, preferably about pH 5.0;
for example, the liquid formulation comprises
(i) about 1-10 mg/mL of the immunoconjugate;
(ii) about 10-30 mM of histidine;
(iii) about 70-90 mg/mL of sucrose, or a combination of about 50-80 mg/mL of sucrose and about 40-60 mM of methionine; and
(iv) about 0.2-0.4 mg/mL of polysorbate 80;
wherein the pH of the liquid formulation is about 5.0-5.5, preferably about pH 5.0;
or, the liquid formulation comprises
(i) about 1.0 mg/mL of the immunoconjugate;
(ii) about 10 mM of histidine;
(iii) about 80 mg/mL of sucrose, or a combination of about 70 mg/mL of sucrose and about 50 mM of methionine, and
(iv) about 0.3 mg/mL of polysorbate 80;
wherein the pH of the liquid formulation is about 5.0-5.5, preferably about pH 5.0;
or, the liquid formulation comprises
(i) about 1.0 mg/mL of the immunoconjugate;
(ii) about 0.12 mg/mL of histidine and about 1.94 mg/mL of histidine hydrochloride;
(iii) about 70 mg/mL of sucrose and about 7.49 mg/mL of methionine, and
(iv) about 0.3 mg/mL of polysorbate 80;
wherein the pH of the liquid formulation is about 5.0-5.5, preferably about pH 5.0.

14. The liquid formulation according to any one of claims 1-13, **characterized in that** the formulation is stable after storage, for example after storage at 2-8°C for at least 3 months, or after storage at room temperature for at least 3 months, or after storage at 40°C±2°C for 1 month, and preferably exhibits one or more of the following characteristics:
(i) as measured by SEC-HPLC, the formulation has a purity of greater than 90%, 95%, 96%, or 97%;
(ii) as measured by non-reduced CE-SDS, the formulation has a purity of greater than 90%, 92%, 94%, 96%, or 98%;
(iii) as measured by iCIEF, the total change in charge heterogeneity (main species, acidic species, and basic species) in the formulation does not exceed 50%, for example does not exceed 40%, 30%, 20%, 10%, or 5%, relative to the initial value at Day 0 of storage; and
(iv) as measured by HPLC-FLD, the variation in polysorbate 80 content in the formulation does not exceed 0.3 mg/mL, for example does not exceed 0.2 mg/mL, 0.10 mg/mL, or 0.05 mg/mL, relative to the initial value at Day 0 of storage.

15. A solid formulation obtained by solidifying the liquid formulation according to any one of claims 1-14, wherein the solid formulation is, for example, in the form of a lyophilized powder for injection.

16. A pharmaceutical injection container comprising the liquid formulation according to any one of claims 1-14 or the solid formulation according to claim 15.

17. A prefilled syringe comprising the liquid formulation according to any one of claims 1-14 or the solid formulation according to claim 15, for intravenous or intramuscular injection.

18. Use of the liquid formulation according to any one of claims 1-14 or the solid formulation according to claim 15, for preventing or treating cancer or for preparing a medicament for preventing or treating cancer, preferably, the cancer is a solid tumor or a hematologic tumor, such as gastrointestinal tumor or melanoma, for example, colorectal cancer or colon cancer; for example, the cancer is a PD-1 antibody therapy-resistant cancer.
